(19) Europäisches Patentamt
European Patent Office
Office européen des brevets

(11) EP 3 691 779 B1

# (12) EUROPEAN PATENT SPECIFICATION

(45) Date of publication and mention of the grant of the patent:
**07.06.2023 Bulletin 2023/23**

(21) Application number: **18779734.5**

(22) Date of filing: **08.10.2018**

(51) International Patent Classification (IPC):
***B01J 13/06*** *(2006.01)* ***A61K 9/127*** *(2006.01)*
***B01J 13/08*** *(2006.01)* ***A61K 47/34*** *(2017.01)*

(52) Cooperative Patent Classification (CPC):
**B01J 13/08; A61K 9/1075; A61K 47/34;**
A61K 9/5146

(86) International application number:
**PCT/EP2018/077365**

(87) International publication number:
**WO 2019/068936 (11.04.2019 Gazette 2019/15)**

## (54) USE OF A COPOLYMER FOR THE STABILISATION OF AN EMULSION

VERWENDUNG EINES COPOLYMERS ZUR STABILISIERUNG EINER EMULSION

UTILISATION D'UN COPOLYMÈRE POUR LA STABILISATION D'UNE ÉMULSION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.10.2017 EP 17306354**

(43) Date of publication of application:
**12.08.2020 Bulletin 2020/33**

(73) Proprietors:
- **Université de Bordeaux**
  **33000 Bordeaux (FR)**
- **Institut Polytechnique de Bordeaux**
  **33400 Talence (FR)**
- **Centre National de la Recherche Scientifique**
  **75016 Paris (FR)**
- **Fundación Cidetec**
  **20014 Donostia-San Sebastián (ES)**

(72) Inventors:
- **LECOMMANDOUX, Sébastien**
  **33610 Canejan (FR)**
- **DUPIN, Damien**
  **20014 DONOSTIA-SAN SEBASTIAN (ES)**
- **LOINAZ BORDONABE, Iraida**
  **20014 DONOSTIA-SAN SEBASTIAN (ES)**
- **GRANDE TELLERIA, Hans-Jürgen**
  **20014 DONOSTA-SAN SEBASTIAN (ES)**
- **LOPEZ ELORZA, Aitziber**
  **20014 DONOSTIA-SAN SEBASTIAN (ES)**
- **GARANGER, Elisabeth**
  **33400 Talence (FR)**

(74) Representative: **ZBM Patents - Zea, Barlocci & Markvardsen**
**Rambla Catalunya, 123**
**08008 Barcelona (ES)**

(56) References cited:
**EP-A1- 2 433 618** **EP-A1- 2 554 161**
**US-A1- 2004 138 095**

- **LV SHIXIAN ET AL: "Co-delivery of doxorubicin and paclitaxel by PEG-polypeptide nanovehicle for the treatment of non-small cell lung cancer", BIOMATERIALS, vol. 35, no. 23, 1 May 2014 (2014-05-01), pages 6118-6129, XP028664977, ISSN: 0142-9612, DOI: 10.1016/J.BIOMATERIALS.2014.04.034**
- **SHIXIAN LV ET AL: "Doxorubicin-loaded amphiphilic polypeptide-based nanoparticles as an efficient drug delivery system for cancer therapy", ACTA BIOMATERIALIA, vol. 9, no. 12, 17 August 2013 (2013-08-17), pages 9330-9342, XP055520437, AMSTERDAM, NL ISSN: 1742-7061, DOI: 10.1016/j.actbio.2013.08.015**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- ZAHEER AHMAD ET AL: "-Phenylalanine) Nanoparticles against Human Breast Cancer Cell : Cisplatin Loaded mPEG- b -P(Glu- co -Phe) Nanoparticles", MACROMOLECULAR BIOSCIENCE, vol. 14, no. 9, 16 June 2014 (2014-06-16), pages 1337-1345, XP055520444, DE ISSN: 1616-5187, DOI: 10.1002/mabi.201400109
- CHENGUANG YANG ET AL: "-phenylalanine)", BIOMATERIALS SCIENCE, vol. 6, no. 8, 27 June 2018 (2018-06-27), pages 2189-2196, XP055520424, GB ISSN: 2047-4830, DOI: 10.1039/C8BM00506K
- CHEN PEIPEI ET AL: "-glutamic acid) Triblock Copolymer for Efficient Loading and Active Intracellular Delivery of Doxorubicin Hydrochloride", BIOMACROMOLECULES, [Online] vol. 16, no. 4, 19 March 2015 (2015-03-19) , pages 1322-1330, XP055795693, US ISSN: 1525-7797, DOI: 10.1021/acs.biomac.5b00113 Retrieved from the Internet: URL:https://pubs.acs.org/doi/pdf/10.1021/acs.biomac.5b00113> [retrieved on 2021-04-15]

## Description

### Technical field

The present invention concerns a new pH-responsive polymeric emulsifier.

### Background Art

Copolymers based on poly(ethylene oxide)-poly(propylene glycol) are widely used to stabilize oil-in-water or water-in-oil emulsions. Varying the degree of polymerization and/or the architecture of the block copolymer, i.e. di or tri-block, allows the hydrophilic-Lipophilic balance to be adjusted. However, none of these copolymers are pH-responsive macro emulsifiers.

Shixian et at disclosed the preparation of methoxy(ethylene glycol)-b-poly(L-glutamic acid)-b-poly(L-lysine)-deoxycholate (DOCA) micelles loaded with the active ingredients doxorubicin or paclitaxel by mixing the drug and the copolymer in polar solvents such as water or dimethylsulphoxide (DMSO) and with the subsequent dialysis or addition of a phosphate buffer. However, it is not disclosed the formation of an emulsion. (D1: LV SHIXIAN ET AL: "Co-delivery of doxorubicin and paclitaxel by PEG-polypeptide nanovehicle for the treatment of non-small cell lung cancer", Biomaterials, 2014, vol. 35(23), pp. 6118-6129).

Some recent studies have been dealing with the use of polypeptides for the stabilization of emulsions. For example, poly(glutamic acid) PGlu homopolymer with Mw between 50,000 and 100,000 g $mol^{-1}$ was used for the stabilization of emulsions of vegetable oils at room temperature (J. Am. Chem. Soc. 2006, 128, 6540-6541). Stable oil-in-water emulsions were obtained at pH > 5.

Poly(L-lysine.HBr)$_x$-b-poly(*racemic*-leucine)$_y$ and poly(L-lysine.HBr)$_x$-b-poly(L-leucine)$_y$ with polyleucine block as permanent hydrophobic block were used as macroemulsifiers to produce double emulsions and only oil-in-water emulsions, respectively. Especially, the article of Nature, 2008, 455, 85 (DOI : 10.1038/nature07197) deals with the production of water-in-oil-in-water double emulsions due to the affinity of poly(L-lysine.HBr)$_x$-b-poly(racemic-leucine)$_y$ with both phases but does not relate to any pH-responsive macroemulsifier.

To encapsulate active ingredients or drugs, loads of systems have been developed.

However, there are only few examples of such encapsulation technology that can be triggered to induce the release of the encapsulated compound.

For food encapsulation, alginate based capsules have been used to protect relevant compounds from the acid environment encountered in the stomach. Other synthetic polymers based on degradable polyesters have also been used. With those polymers, the release is dictated by the degradation of the polymer, resulting in the continuous release of the compound and not a burst release.

To date, there is still a need for a system such as capsules allowing a triggered release of active compounds.

### Summary of invention

The aim of the present invention is to provide a pH-responsive polymeric macroemulsifier. The aim of the present invention is also to provide a polymeric emulsifier able to allow the encapsulation of hydrophobic or hydrophilic active compounds.

Thus, the present invention relates to the use of a copolymer having the following formula (II):

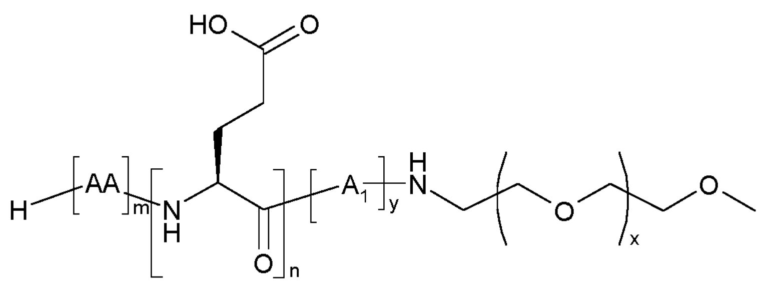

(II)

wherein:

- $A_1$ is a linker, preferably an enzyme cleavable peptide sequence, and y is 0 or 1;
- AA is a residue of a hydrophobic amino acid, such as valine, leucine, isoleucine, or phenylalanine;
- x is an integer comprised between 17 and 270, and preferably between 17 and 114;
- n is an integer comprised between 5 and 170; and
- m is an integer comprised between 0 and 80,

optionally in the presence of a co-surfactant,
as surfactant for the stabilization of an emulsion at a pH comprised between 4 and 6.5, preferably between 5 and 6.

## Detailed description of the invention

Capsules disclosed herein above and below, the preparation process thereof, embodiments thereof and examples thereof are not forming part of the present invention.

Compositions comprising at least one capsules as disclosed herein above and below, the preparation process thereof, embodiments thereof, and examples thereof are not forming part of the present invention. Water-in-oil emulsions comprising in the dispersed aqueous phase at least one capsule as defined above and below, the preparation process thereof, embodiments thereof and examples thereof are not forming part of the present invention. Oil-in-water emulsion comprising in dispersed fatty phase at least one capsule as defined above and below, the preparation process thereof, embodiments thereof and examples thereof are not forming part of the present invention. A glycerol-in-oil emulsion comprising a dispersed glycerol phase comprises at least one capsule as defined above and below, the preparation process thereof, embodiments thereof and examples thereof are not forming part of the present invention. An Oil-in-glycerol emulsion comprising a dispersed fatty phase comprises at least one capsule as defined above and below, the preparation process thereof, embodiments thereof and examples thereof are not forming part of the present invention. Methods for the encapsulation of a hydrophobic active compound, embodiments thereof and examples thereof are not forming part of the present invention. Methods for the release of at least one active compound comprising the addition of a capsule disclosed herein above or below, embodiments thereof and examples thereof are not forming part of the present invention.

The copolymers comprise a PEG block comprising Glu and AA amino acids, as well as the linker $A_1$. According to an embodiment, the copolymers are diblock copolymers (one block comprising amino acids and one block comprising PEG groups).

According to an embodiment, in formula (II), the repeating units AA and Glu are copolymerized randomly.

Within the present application, the copolymers may also be represented as $P(Glu_n\text{-co-}(AA)_m\text{-}b\text{-}(A_1)_y\text{-}b\text{-}PEG_x$ or $P(Glu_n\text{-co}(AA)_m)\text{-}(A_1)_y\text{-}PEG_x$ indifferently.

The present invention also relates to the use of a copolymer having the following formula (II):

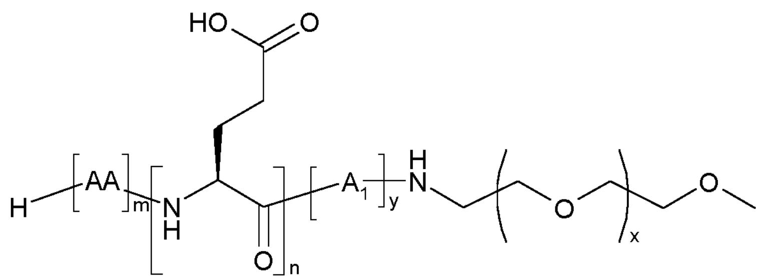

(II)

wherein:

- $A_1$ is a linker, preferably an enzyme cleavable peptide sequence, and y is 0 or 1;
- AA is a residue of a hydrophobic amino acid, such as valine, leucine, isoleucine, or phenylalanine;
- x is an integer comprised between 17 and 270, and preferably between 17 and 114;
- n is an integer comprised between 5 and 170; and
- m is an integer comprised between 0 and 80,

as surfactant for the stabilization of an emulsion at a pH comprised between 4 and 6.5, preferably between 5 and 6.

The present invention also relates to the use of a copolymer having the following formula (II):

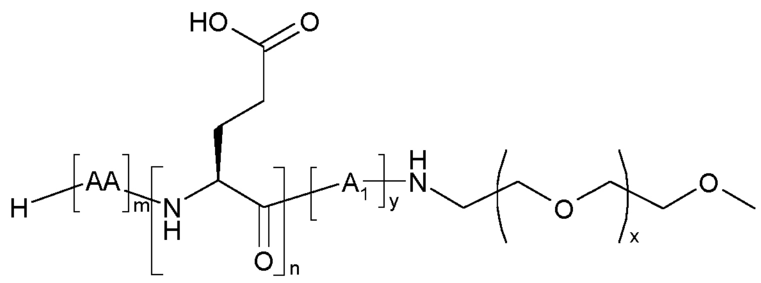

(II)

wherein:

- $A_1$ is a linker, preferably an enzyme cleavable peptide sequence, and y is 0 or 1;
- AA is a residue of a hydrophobic amino acid, such as valine, leucine, isoleucine, or phenylalanine;
- x is an integer comprised between 17 and 270, and preferably between 17 and 114;
- n is an integer comprised between 5 and 170; and
- m is an integer comprised between 0 and 80,

in the presence of a co-surfactant,
as surfactant for the stabilization of an emulsion at a pH comprised between 4 and 6.5, preferably between 5 and 6.

The present invention also relates to the use of a copolymer having the following formula (II):

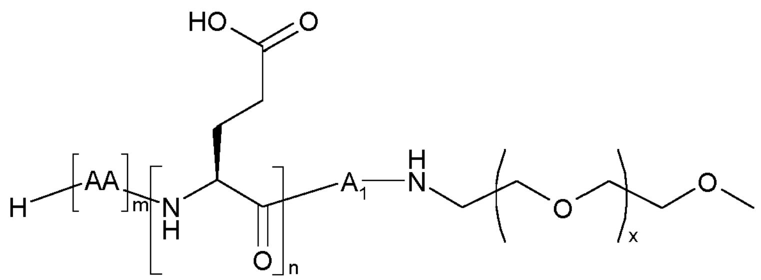

(II)

wherein:

- $A_1$ is a linker,
- AA is a residue of a hydrophobic amino acid, such as valine, leucine, isoleucine, or phenylalanine;
- x is an integer comprised between 17 and 270, and preferably between 17 and 114;
- n is an integer comprised between 5 and 170; and
- m is an integer comprised between 0 and 80,

as surfactant for the stabilization of an emulsion at a pH comprised between 4 and 6.5, preferably between 5 and 6.

The present invention also relates to the use of a copolymer having the following formula (I):

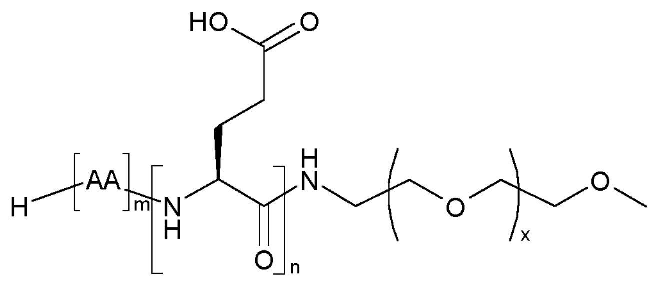

(I)

which also writes as:

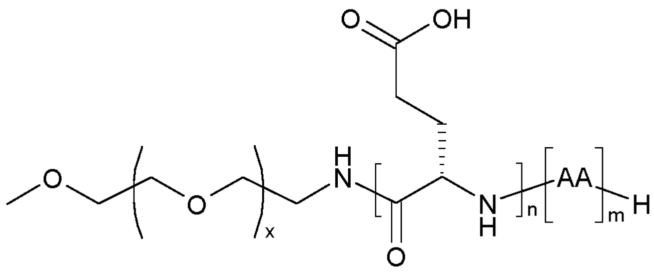

(I)

wherein:

- AA is a residue of a hydrophobic amino acid, such as valine, leucine, isoleucine, or phenylalanine;
- x is an integer comprised between 17 and 270, and preferably between 17 and 114;
- n is an integer comprised between 5 and 160; and
- m is an integer comprised between 0 and 80,

in the presence of a co-surfactant,
as surfactant for the stabilization of an emulsion at a pH comprised between 4 and 6.5, preferably between 5 and 6.

Indeed, the copolymer as defined above, of formula (I) or (II), has interesting pH-responsive emulsifying properties and can be used as a polymeric emulsifier depending on the pH of the emulsion.

The copolymer of formula (I) or (II) may stabilize an emulsion having a pH comprised between 4 and 6.5. Indeed, emulsions comprising said copolymer are stable over time for several months.

Outside this pH range, this copolymer is not able to stabilize any emulsion at long term, i.e. demulsification occurred in less than 1 week.

The copolymer of formula (II) may comprise a linker $A_1$ as defined above. Preferably, the linker $A_1$ is a peptide linker.

Preferably, $A_1$ is an enzyme-cleavable peptide sequence, in particular a peptide sequence cleavable by matrix metalloproteinase (MMP), in particular by MMP2 and MMP9. According to a preferred embodiment, $A_1$ is a linker being a peptide comprising from 4 to 10 amino acids, preferably from 4 to 7 amino acids, and containing in particular at least one glycine residue, and preferably at least two glycine residues, and optionally at least one additional amino acid in the β configuration. According to an embodiment, $A_1$ may also comprise at least one additional amino acid in the β configuration, preferably at the N or the C terminal end of the peptide. According to a preferred embodiment, $A_1$ includes beta-alanine (βA).

According to a preferred embodiment, $A_1$ is a linker containing a peptide comprising from 4 to 10 amino acids, and at least one further amino acid in the β configuration. According to a preferred embodiment, $A_1$ is chosen from the group consisting of the following peptides: βA-PVGLiG, βA-GFLG, βA-GRFG, and βA-GFKFLG. More preferably, $A_1$ is the peptide of formula βA-PVGLIG.

In one embodiment, the copolymer of formula (I) or (II) is used in combination with a co-surfactant.

Preferably, the co-surfactant is chosen from the group consisting of co-surfactants with a hydrophilic-lipophilic balance (HLB) below 10 and preferably between 3 and 7. According to the invention, HLB is for a surfactant a measure of the degree to which it is hydrophilic or hydrophobic, determined by calculating values for the different regions of the molecule, as described by Griffin.

Griffin's method for non-ionic surfactants is described as follows:

$$HLB = 20 * M_h/M$$

where $M_h$ is the molecular mass of the hydrophilic portion of the molecule, and M is the molecular mass of the whole molecule, giving a result on a scale of 0 to 20. An HLB value of 0 corresponds to a completely hydrophobic molecule, and a value of 20 corresponds to a completely hydrophilic molecule.

According to a preferred embodiment, the co-surfactant is chosen from the nonionic emulsifiers, such as oxyalkylenated (more particularly polyoxyethylenated) fatty acid esters of glycerol; oxyalkylenated fatty acid esters of sorbitan; oxyalkylenated (oxyethylenated and/or oxypropylenated) fatty acid esters; oxyalkylenated (oxyethylenated and/or oxypropylenated) fatty alcohol ethers; sugar esters such as sucrose stearate; and mixtures thereof, such as the mixture of glyceryl stearate and PEG-40 stearate, as well as esters of sugars, such as sucrose stearate; or ethers of fatty alcohol

and of sugar, in particular alkyl polyglucosides (APGs).

As alkyl- and polyalkyl- esters of sorbitan, polyoxyethylenated or not, those having a number of ethylene oxide (EO) patterns ranging from 0 to 100 are preferably used. Mention can for example be made of sorbitan laurate 4 or 20 OE, in particular polysorbate 20 (or polyoxyethylene (20) sorbitan monolaurate), sorbitan palmitate 20 OE, sorbitan stearate 20 OE or sorbitan oleate 20 OE. Mention can also be made of sorbitan oleate, such as the product sold under the trade name Massocare SMO, or sorbitan palmitate, such as the product sold under the trade name Massocare SMP, or sorbitan stearate.

As alkyl- and polyalkyl- esters of glycerol, polyoxyethylenated or not, those having a number of ethylene oxide (EO) patterns ranging from 0 to 100 and a number of glycerol patterns ranging from 1 to 30 are preferably used. Mention can for example be made of PEG-150 distearate; hexaglyceryl monolaurate; or the mixture of glyceryl stearate and of PEG-100 stearate.

The preferred co-surfactants are selected from the group consisting of: Polyglyceryl-3 Sorbityl Linseedate (such as the product sold under the trade name Ewocream® by Sinerga), sorbitan oleate (such as the product sold under the trade name Massocare SMO), sorbitan palmitate (such as the product sold under the trade name Massocare SMP), sorbitan stearate, and polglyceryl-10 decaoleate (such as the product sold under the trade name Polyaldo® 10-10-0), and mixtures thereof.

It is disclosed but not being part of the present invention a capsule having a diameter comprised between 50 nm and 500 μm, comprising a core comprising or consisting of one single droplet of water, glycerol or of oil,
wherein said core is surrounded by one polymeric shell which encapsulates totally said single droplet, and said polymeric shell consists or substantially consists of a possibly crosslinked copolymer having the formula (II) as defined above.

Within the present application, the expression "possibly crosslinked copolymer" means that said copolymer is a copolymer which is either crosslinked or not. Thus, in one embodiment not being part of the present invention the "possibly crosslinked copolymer" means the copolymer of formula (I) or (II). Alternatively, in another embodiment not being part of the present invention, the "possibly crosslinked copolymer" means a crosslinked copolymer of formula (I) or (II).

According to an embodiment not being part of the present invention, the polymeric shell of the capsule consists of a copolymer having the formula (II), said copolymer being crosslinked or not. According to an embodiment not being part of the present invention, the polymeric shell of the capsule consists of a crosslinked copolymer having the formula (II).

According to an embodiment not being part of the present invention, the polymeric shell of the capsule consists of a copolymer having the formula (I), said copolymer being crosslinked or not. According to an embodiment not being part of the present invention, the polymeric shell of the capsule consists of a crosslinked copolymer having the formula (I). According to an embodiment not being part of the present invention, the polymeric shell of the capsule consists of a non-crosslinked copolymer having the formula (I).

According to an embodiment, it is disclosed but not being part of the present invention, a capsule having a diameter comprised between 50 nm and 500 μm, comprising a core comprising one single droplet of water, glycerol or of oil,
wherein said core is surrounded by one polymeric shell which encapsulates totally said single droplet, and said polymeric shell consists of a copolymer having the formula (II) as defined above.

It is disclosed but not being part of the present invention a capsule having a diameter comprised between 50 nm and 500 μm, comprising a core comprising or consisting of one single droplet of water or glycerol or of oil,
wherein said core is surrounded by one polymeric shell which encapsulates totally said single droplet, and said polymeric shell consists or substantially consists of a possibly crosslinked copolymer having the following formula (I):

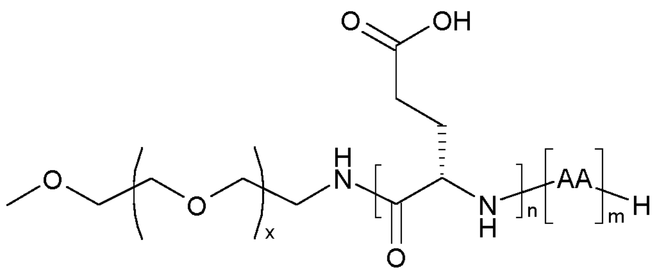

(I)

wherein:

- AA is a residue of a hydrophobic amino acid, such as valine, leucine, isoleucine, or phenylalanine;
- x is an integer comprised between 17 and 270, and preferably between 17 and 114;

- n is an integer comprised between 5 and 160; and
- m is an integer comprised between 0 and 80.

According to an embodiment, it is disclosed but not being part of the present invention, a capsule having a diameter comprised between 50 nm and 500 μm, comprising a core comprising one single droplet of water or glycerol or of oil,
wherein said core is surrounded by one polymeric shell which encapsulates totally said single droplet, and said polymeric shell consists of a copolymer having the formula (I) as defined above.

The capsules not being part of the invention can also be designated as nanocapsules depending on their size.

The capsules not being part of the invention have a core-shell structure. As mentioned above, they comprise a core which is made of water, glycerol, or oil, surrounded by a polymeric shell made of the polymer of formula (I) or (II) as defined above.

The size of the capsules not being part of the invention corresponds to their diameter. This diameter may be measured by any of the following methods:

- By Dynamic Light Scattering: Hydrodynamic diameters were measured at 25°C using a Malvern Zetasizer NanoZS instrument equipped with a 4 mW He-Ne solid-state laser operating at 633 nm. Back-scattered light was detected at 173° and the mean particle diameter was calculated from the quadratic fitting of the correlation function over thirty runs of ten seconds duration. All measurements were performed three times on 0.01 w/v % aqueous latex solutions. The pH of the deionized water used to dilute the latex was matched to that of the capsules dispersion (pH 5.5) based on oil-in-water emulsions and was ultra-filtered through a 0.20 μm membrane so as to remove any dust.

- Or: A Malvern Mastersizer 2000 or 3000 instrument equipped with a small volume Hydro 2000SM sample dispersion unit (ca. 50 mL), a HeNe laser operating at 633 nm, and a solid-state blue laser operating at 466 nm was used to size the emulsions at pH 10. The stirring rate was adjusted to 1000 or 2000 rpm in order to avoid creaming of the emulsion during analysis. The mean droplet diameter was taken to be the volume mean diameter (D4/3), which is mathematically expressed as D4/3 = ΣDi4Ni/ΣDi3Ni. The standard deviation for each diameter provides an indication of the width of the size distribution. After each measurement, the cell was rinsed once with ethanol, followed by three rinses using water. The glass walls of the cell were carefully wiped with lens cleaning tissue to avoid cross-contamination, and the laser was aligned centrally on the detector. This setup allowed continuous measurements to be made after the sample chamber pH had been adjusted from 10 to 3. This allowed droplet stability to be examined with regard to any changes in pH.
- Or: A drop of the diluted emulsion was placed on a microscope slide and viewed using an optical microscope (James Swift MP3502, Prior Scientific Instruments Ltd.) connected to a PC laptop to record images. This technique was used to estimate the mean droplet diameter. The response of the o/w emulsion droplets following in situ acidification of the aqueous phase was also assessed using this equipment.

Preferably, the diameter of the capsules may be measured by Dynamic Light Scattering (DLS) or Laser Diffraction, both methods giving the same results. Most preferably, the diameter of the capsules is measured by Dynamic Light Scattering (DLS).

Within the present application, the values given for the size of the capsules are obtained by measuring the size by DLS.

According to the invention but not being part of it, the expression "capsules" means that the core-shell structure as defined above is not result of a self-assembly phenomenon like in the case of micelles and vesicles where the diblock copolymer is forming spontaneously those objects due to the hydrophobic character of the polypeptide block at those pH range. In the current invention but not being part of it, capsules require sufficient energy to form droplet of water or glycerol or oil into its corresponding immiscible phase, i.e. oil, or water, or glycerol, respectively.

According to the invention but not being part of it, the energy required to form the droplet can be applied by high shear mixing, sonication, or high-pressure homogenization process.

According to the invention but not being part of it, the expression "substantially consists of" means that the polymeric shell may comprise other compounds than the crosslinked copolymer of formula (I) or (II) as defined above. However, these compounds preferably do not have any structuring effect.

***Core***

According to an embodiment not being part of the invention, the core of the capsules comprises or consists of one single droplet of water.

According to an embodiment not being part of the invention, the core of the capsules comprises or consists of

one single droplet of glycerol.

According to another embodiment not being part of the invention, the core of the capsules comprises or consists of one single droplet of oil.

Preferably, the oil is chosen from the group consisting of vegetable oils, and cosmetic oils such as octyl palmitate, caprylic/capric triglyceride, isodecyl isononanoate, isohexadecane, squalene (phytosqualene), *Simmondsia Chinensis* (Jojoba) seed oil and silicone-based oil.

The oil may also be any water-immiscible organic solvent such as chloroform or dichloromethane.

More preferably, the oil is dodecane, olive oil, octyl palmitate or sunflower oil.

According to an embodiment not being part of the invention, the core of the capsules further comprises at least one active compound.

As active compound, one may cite the followings: drugs, vitamins, antioxidants, natural ingredients, natural extracts, peptides, foods, natural or hydrophilic polymers (such as hyaluronic acid), and mixtures thereof. The active compound may be hydrophilic or hydrophobic.

As preferred active compounds, the followings may be mentioned: retinyl palmitate and retinol as precursor of Vitamin A, Curcumin, α-tocopherol as precursor of Vitamin E, ceramides for hydrophobic compounds to be dissolved in the oil phase for O/W system and dipotassium Glycyrrhizate and hyaluronic acid to be dissolved in the water phase for W/O system.

Preferably, the active compounds are selected from the followings: *Curcuma Longa* root extract, Ceramide NG, retinyl palmitate, dipotassium glycyrrhizate, and hyaluronic acid.

***Polymeric shell***

The polymeric shell of the capsules is made of a copolymer as defined above which can also be designated by the formula

$P(Glu_n\text{-}AA_m)\text{-}(A_1)_y\text{-}PEG_x$, AA, $A_1$, y, x, m, and n being as defined above. PEG refers to poly(ethylene glycol).

The copolymer of formula (I) or (II) thus comprises a PEG block and a polypeptide block. The polypeptide block comprises Glu and possibly AA units, said units being randomly distributed.

AA is a hydrophobic amino acid, and is preferably valine, leucine, isoleucine, or phenylalanine, in particular AA is valine.

According to an embodiment, in formula (I) or (II) as defined above, m is an integer comprised between 0 and 40, and is preferably 0.

According to an embodiment, the copolymer used according to the invention has the following formula (I-1):

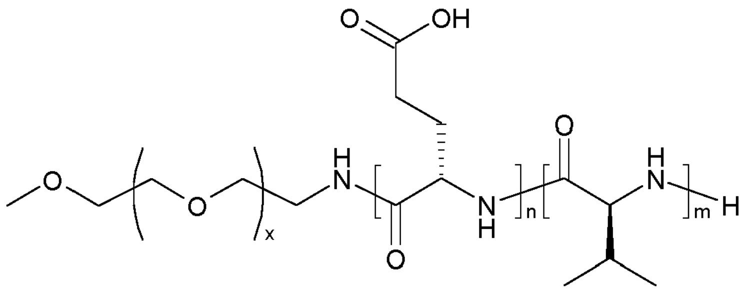

(I-1)

wherein x, n, and m are as defined above in formula (I).

Copolymers of formula (I-1) may also be represented as follows:

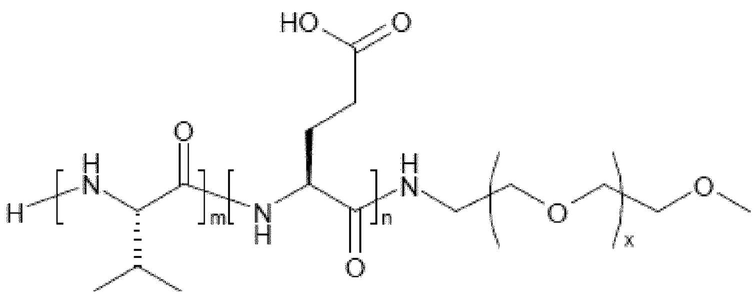

According to an embodiment, in formula (I) or (I-1) as defined above, m is 0.

According to an embodiment, in formula (II) as defined above, m is 0.

Preferably, the polymeric shell of the capsules consists of a copolymer of formula (I), wherein x is an integer comprised between 40 and 120, preferably between 44 and 114, and most preferably is 44 or 114.

Preferably, the polymeric shell of the capsules consists of a copolymer of formula (I), wherein n is an integer comprised between 10 and 160, preferably between 15 and 155, more preferably between 15 and 151, and most preferably is 15, 16, 25, 36, 46, 47, 58, 96, and 151.

Preferably, the polymeric shell of the capsules consists of a copolymer of formula (I), wherein m is 0 or is an integer comprised between 5 and 15, and is preferably 0, 5, 7 or 11.

Preferably, the polymeric shell of the capsules consists of a copolymer of formula (II), wherein x is an integer comprised between 40 and 120, preferably between 44 and 114, and most preferably is 44 or 114, y=1 and $A_1$ is βA-PVGLIG.

Preferably, the polymeric shell of the capsules consists of a copolymer of formula (II), wherein n is an integer comprised between 10 and 170, preferably between 60 and 170, and most preferably is 61, 100, 150, and 170, y=1 and $A_1$ is βA-PVGLIG.

Preferably, the polymeric shell of the capsules consists of a copolymer of formula (II), wherein m is 0 or is an integer comprised between 5 and 20 and is preferably 0 or 16.

Preferably, the polymeric shell of the capsules consists of a copolymer of formula (I), wherein:

- x is an integer comprised between 40 and 120, preferably between 44 and 114, and most preferably is 44, or 114,
- m is 0, and
- n is an integer comprised between 10 and 160, preferably between 15 and 155, more preferably between 15 and 151, and most preferably is 15, 16, 25, 36, 46, 47, 58, 96, and 151.

Preferably, the polymeric shell of the capsules consists of a copolymer of formula (I) wherein m is 0, n is 96, 100, or 151 and x is 114, or wherein m is 5, n is 16 and x is 114 or wherein m is 11, n is 44 and x is 114 or wherein m is 11, n is 46 and x is 114.

Preferably, the polymeric shell of the capsules consists of a copolymer of formula (II), wherein:

- y=1 and $A_1$ is βA-PVGLIG;
- x is an integer comprised between 40 and 120, preferably between 44 and 114, and most preferably is 114,
- m is 0, and
- n is an integer comprised between 60 and 170, and most preferably is 100, 150, and 170.

Preferably, the polymeric shell of the capsules consists of a copolymer of formula (II) wherein y=1, $A_1$ is βA-PVGLIG, m is 0, n is 100, 150, or 170 and x is 114, or wherein y=1, $A_1$ is βA-PVGLIG, m is 16, n is 61 and x is 114, and wherein y=1, $A_1$ is βA-PVGLIG, m is 11, n is 46 and x is 114.

More preferably, the polymeric shell of the capsules consists of a copolymer having one of the following formulae:

$PEG_{44}$-$PGlu_{25}$, which may also be written as $PEG_{44}$-*b*-$PGlu_{25}$;
$PEG_{44}$-$PGlu_{36}$, which may also be written as $PEG_{44}$-*b*-$PGlu_{36}$;
$PEG_{44}$-$PGlu_{47}$, which may also be written as $PEG_{44}$-*b*-$PGlu_{47}$;
$PEG_{44}$-$PGlu_{58}$, which may also be written as $PEG_{44}$-*b*-$PGlu_{58}$;
$PEG_{44}$-$PGlu_{100}$, which may also be written as $PEG_{44}$-*b*-$PGlu_{100}$;
$PEG_{114}$-$PGlu_{151}$, which may also be written as $PEG_{114}$-*b*-$PGlu_{151}$;
$PEG_{114}$-$PGlu_{96}$, which may also be written as $PEG_{114}$-*b*-$PGlu_{96}$;
$PEG_{114}$-P($Glu_{46}$-$Val_{11}$), which may also be written as $PEG_{114}$-P($Glu_{46}$-co-$Val_{11}$) or $PEG_{114}$-*b*-P($Glu_{46}$-co-$Val_{11}$);
$PEG_{114}$-P($Glu_{51}$-$Val_{6}$), which may also be written as $PEG_{114}$-P($Glu_{51}$-co-$Val_{6}$) or $PEG_{114}$-*b*-P($Glu_{51}$-co-$Val_{6}$);
$PEG_{44}$-P($Glu_{15}$-$Val_{7}$), which may also be written as $PEG_{44}$-P($Glu_{15}$-co-$Val_{7}$) or $PEG_{44}$-*b*-P($Glu_{15}$-co-$Val_{7}$);
$PEG_{114}$-P($Glu_{16}$-$Val_{5}$), which may also be written as $PEG_{114}$-P($Glu_{16}$-co-$Val_{5}$) or $PEG_{114}$-*b*-P($Glu_{16}$-co-$Val_{5}$);
$PEG_{114}$-P($Glu_{46}$-$Val_{11}$), which may also be written as $PEG_{114}$-P($Glu_{46}$-co-$Val_{11}$) or $PEG_{114}$-*b*-P($Glu_{46}$-co-$Val_{11}$);

More preferably, the polymeric shell of the capsules consists of a copolymer (II) having one of the following formulae:

$PGlu_{150}$-βA-PVGLIG-$PEG_{114}$, which may also be written as $PGlu_{150}$-*b*-βA-PVGLIG-$PEG_{114}$;
$PGlu_{100}$-βA-PVGLIG-$PEG_{114}$, which may also be written as $PGlu_{100}$-*b*-βA-PVGLIG-$PEG_{114}$;
$PGlu_{170}$-βA-PVGLIG-$PEG_{114}$, which may also be written as $PGlu_{170}$-*b*-βA-PVGLIG-$PEG_{114}$;

$P(Glu_{46}-Val_{11})$-βA-PVGLIG-$PEG_{114}$, which may also be written as $P(Glu_{46}$-co-$Val_{11})$-βA-PVGLIG-$PEG_{114}$ or $P(Glu_{46}$-co-$Val_{11})$-*b*-βA-PVGLIG-*b*-$PEG_{114}$ and

$P(Glu_{61}-Val_{16})$-βA-PVGLIG-$PEG_{114}$, which may also be written as $P(Glu_{61}$-co-$Val_{16})$-βA-PVGLIG-$PEG_{114}$ or $P(Glu_{61}$-co-$Val_{16})$-*b*-βA-PVGLIG-*b*-$PEG_{114}$

According to an embodiment not being part of the invention, the polymeric shell of the capsules as defined above may be crosslinked. In particular, the Glu units may be crosslinked via amidation using a diamine compound like ethylene diamine or bis(hexamethylene) triamine, but not restricted to.

According to an embodiment not being part of the invention, the capsules comprise a single droplet of water, possibly comprising a hydrophilic active compound, surrounded by a polymeric shell made of a copolymer of formula (I), (II) or (I-1) as defined above. According to an embodiment not being part of the invention, the capsules comprise a single droplet of oil, possibly comprising a hydrophobic active compound, surrounded by a polymeric shell made of a copolymer of formula (I), (II) or (I-1) as defined above.

It is disclosed but not being part of the present invention a composition comprising at least one capsule as defined above.

It is also disclosed but not being part of the present invention a composition comprising one or several capsule(s) as defined above, said capsule comprising a single droplet of water, possibly comprising a hydrophilic active compound, surrounded by a polymeric shell made of a copolymer of formula (I), (II) or (I-1) as defined above.

It is also disclosed but not being part of the present invention a composition comprising one or several capsule(s) as defined above, said capsule comprising a single droplet of glycerol, possibly comprising a hydrophilic active compound, surrounded by a polymeric shell made of a copolymer of formula (I), (II) or (I-1) as defined above.

It is also disclosed but not being part of the present invention a composition comprising one or several capsule(s) as defined above, said capsule comprising a single droplet of oil, possibly comprising a hydrophobic active compound, surrounded by a polymeric shell made of a copolymer of formula (I), (II) or (I-1) as defined above.

It is disclosed but not being part of the present invention a water-in-oil emulsion comprising a dispersed aqueous phase and a continuous fatty phase, wherein:

- the continuous fatty phase comprises at least one oil, preferably chosen from the group consisting of: vegetable oils, cosmetic oils such as octyl palmitate, Caprylic/capric triglyceride, Isodecyl isononanoate, Isohexadecane, Squalene (Phytosqualene), or *Simmondsia Chinensis (Jojoba) Seed Oil*), and water-immiscible organic solvents, such as chloroform or dichloromethane, and is in particular octyl palmitate, dodecane, olive oil or sunflower oil, and
- the dispersed aqueous phase comprises at least one capsule as defined above comprising a core consisting of or comprising a single droplet of water.

According to an embodiment, the capsule further comprises a hydrophilic active compound. Preferably, the core of the capsule comprises at least one hydrophilic active compound.

The water-in-oil emulsion to encapsulate a hydrophilic compound can be prepared from 1% to 50% by weight of water using 1% to 20% by weight of the copolymer of formula (I) or (II) based on the aqueous phase. The amount of hydrophilic active compound encapsulated depends on its solubility in the aqueous phase.

According to an embodiment not being part of the present invention, the water-in-oil emulsion also comprises a co-surfactant as defined above. Preferably, the concentration of the co-surfactant in the emulsion is comprised between 1% and 30%, and preferably between 5% and 20%, by weight in relation to the total weight of said emulsion. According to an embodiment not being part of the present invention, the amount of copolymer of formula (I) or (II) to produce water-in-oil emulsion is comprised between 0.1 wt% and 20 wt% based on the total weight of the emulsion, and preferably between 0.2 wt% and 10 wt%, and more preferably between 0.5 wt% and 5 wt%.

According to an embodiment not being part of the present invention, the ratio copolymer:cosurfactant in weight % is 0.95:4.72, 0.9:9, 0.83:16.53, 1:16, 2:16 and preferably 0.83:16.53, 1:16, 2:16.

It is disclosed but not being part of the present invention an oil-in-water emulsion comprising a dispersed fatty phase and a continuous aqueous phase, wherein:

- the continuous aqueous phase comprises water between pH 4 and 6.5, preferably between 5 and 6, and
- the dispersed fatty phase comprises at least one capsule as defined above comprising a core consisting of or comprising a single droplet of oil.

According to an embodiment not being part of the present invention, the capsule further comprises a hydrophobic active compound. Preferably, the core of the capsule comprises at least one hydrophobic active compound.

The oil-in-water emulsion not being part of the present invention to encapsulate the hydrophobic compound can be prepared from 1% to 50% by weight of oil using from 1% to 20% by weight of the copolymer of formula (I) or (II)

based on the oil phase. The amount of encapsulated compound depends on the maximum amount of solubility of the active ingredient in the oil.

According to an embodiment not being part of the present invention, the oil-in-water emulsion also comprises a co-surfactant as defined above. Preferably, the concentration of the co-surfactant in the emulsion is comprised between 1% and 30%, and preferably between 5% and 20%, by weight in relation to the total weight of said emulsion.

It is disclosed but not being part of the present invention a glycerol-in-oil emulsion comprising a dispersed glycerol phase and a continuous fatty phase, wherein:

- the continuous fatty phase comprises at least one oil, preferably chosen from the group consisting of: vegetable oils, cosmetic oils such as octyl palmitate, isodecyl isononanoate, isohexadecane, squalene (Phytosqualene), or *Simmondsia Chinensis (Jojoba) Seed Oil),* and water-immiscible organic solvents, such as chloroform or dichloromethane, and is in particular caprylic/capric triglyceride, octyl palmitate, dodecane, olive oil or sunflower oil, and
- the dispersed glycerol phase comprises at least one capsule as defined above comprising a core consisting of or comprising a single droplet of glycerol.

According to an embodiment not being part of the present invention, the capsule further comprises a hydrophilic active compound. Preferably, the core of the capsule comprises at least one hydrophilic active compound.

According to the invention not being part of the present invention, the glycerol-in-oil emulsion to encapsulate a hydrophilic compound can be prepared from 1% to 50% by weight of glycerol using 1 % to 20% by weight of the copolymer of formula (I) or (II) based on the dispersed phase. The amount of hydrophilic active compound encapsulated depends on its solubility in the dispersed phase.

According to an embodiment not being part of the present invention, the glycerol-in-oil emulsion also comprises a co-surfactant as defined above. Preferably, the concentration of the co-surfactant in the emulsion is comprised between 1% and 30%, and preferably between 5% and 20%, by weight in relation to the total weight of said emulsion. According to an embodiment not being part of the present invention, the ratio copolymer:cosurfactant in weight % is 0.95:4.72, 0.9:9, 0.83:16.53, 1:16, 2:16 and preferably 0.83:16.53, 1:16 and 2:16.

It is disclosed but not being part of the present invention an oil-in-glycerol emulsion comprising a dispersed fatty phase and a continuous phase, wherein:

- the continuous phase comprises glycerol, and
- the dispersed fatty phase comprises at least one capsule as defined above comprising a core consisting of or comprising a single droplet of oil.

According to an embodiment not being part of the present invention, the capsule further comprises a hydrophobic active compound. Preferably, the core of the capsule comprises at least one hydrophobic active compound.

According to the invention not being part of the present invention, the oil-in-glycerol emulsion to encapsulate the hydrophobic compound can be prepared from 1% to 50% by weight of oil using from 1% to 20% by weight of the copolymer of formula (I) or (II) based on the oil phase. The amount of encapsulated compound depends on the maximum amount of solubility of the active ingredient in the oil.

According to an embodiment not being part of the present invention, the oil-in-glycerol emulsion also comprises a co-surfactant as defined above. Preferably, the concentration of the co-surfactant in the emulsion is comprised between 1% and 30%, and preferably between 5% and 20%, by weight in relation to the total weight of said emulsion.

According to an embodiment not being part of the present invention, in the emulsions not being part of the present invention, the amount of copolymer of formula (I) or (II) is comprised between 1% and 10% by weight in relation to the total weight of said emulsion. According to an embodiment not being part of the present invention, in the emulsions not being part of the present invention, the amount of dispersed phase is comprised between 1% and 50% by weight in relation to the total weight of said emulsion.

It is also disclosed but not being part of the present invention, a method for the encapsulation of a hydrophobic active compound, comprising the following steps:

- a step of preparing a fatty phase by dissolving a hydrophobic active compound in a fatty solution comprising at least one oil,
- an optional step of adding a co-surfactant as defined above,
- a step of preparing an aqueous solution at a pH between 4 and 6.5, preferably between 5 and 6, said solution containing a copolymer having the formula (I) or (II) as defined above,
  and
- a step of dispersing said fatty phase into said aqueous solution, in order to obtain a capsule not being part of the present invention having a diameter comprised between 50 nm and 500 μm, comprising a core consisting of or

comprising one single droplet of oil containing said hydrophobic active compound, wherein said core is surrounded by one polymeric shell which encapsulates totally said single droplet, and said polymeric shell consists of the copolymer having the formula (I) or (II), and
- an optional step of recovering the capsule(s) encapsulating at least one hydrophobic active compound, for example by centrifugation or dialysis.

It is also disclosed but not being part of the present invention, a method for the encapsulation of a hydrophobic active compound, comprising the following steps:

- a step of preparing a fatty phase by dissolving a hydrophobic active compound in a fatty solution comprising at least one oil,
- an optional step of adding a co-surfactant as defined above, and
- a step of preparing a glycerol solution containing a copolymer having the formula (I) or (II) as defined above,
- a step of dispersing said fatty phase into said glycerol solution, in order to obtain a capsule not being part of the present invention having a diameter comprised between 50 nm and 500 μm, comprising a core consisting of or comprising one single droplet of oil containing said hydrophobic active compound, wherein said core is surrounded by one polymeric shell which encapsulates totally said single droplet, and said polymeric shell consists of the copolymer having the formula (I) or (II), and
- an optional step of recovering the capsule(s) encapsulating at least one hydrophobic active compound, for example by centrifugation or dialysis.

It is also disclosed but not being part of the present invention, a method for the encapsulation of a hydrophilic active compound, comprising the following steps:

- a step of preparing an aqueous phase at a pH between 4 and 6.5, preferably between 5 and 6, said solution containing a copolymer having the formula (I) or (II) as defined above, and by dissolving a hydrophilic active compound in water,
- a step of preparing a fatty solution comprising at least one oil
- an optional step of adding a co-surfactant as defined above, and
- a step of dispersing said aqueous phase into said fatty solution, in order to obtain a capsule not being part of the present invention having a diameter comprised between 50 nm and 500 μm, comprising a core consisting of or comprising one single droplet of water containing said hydrophilic active compound, wherein said core is surrounded by one polymeric shell which encapsulates totally said single droplet, and said polymeric shell consists of the copolymer having the formula (I) or (II), and
- an optional step of recovering the capsule(s) encapsulating at least one hydrophilic active compound, for example by centrifugation or dialysis.

It is also disclosed but not being part of the present invention, a method for the encapsulation of a hydrophilic active compound, comprising the following steps:

- a step of preparing a glycerol phase by dissolving a hydrophilic active compound in glycerol, said solution containing a copolymer having the formula (I) or (II) as defined above,
- a step of preparing a fatty solution comprising at least one oil ,
- an optional step of adding a co-surfactant as defined above, and
- a step of dispersing said glycerol phase into said fatty solution, in order to obtain a capsule not being part of the present invention having a diameter comprised between 50 nm and 500 μm, comprising a core consisting of or comprising one single droplet of glycerol containing said hydrophilic active compound, wherein said core is surrounded by one polymeric shell which encapsulates totally said single droplet, and said polymeric shell consists of the copolymer having the formula (I) or (II), and
- an optional step of recovering the capsule(s) encapsulating at least one hydrophilic active compound, for example by centrifugation or dialysis.

According to another embodiment not being part of the present invention, the encapsulation of a hydrophilic active compound may be carried out by adding the hydrophilic active compound in water or glycerol in the presence of the capsules not being part of the present invention with the crosslinked copolymer as defined above and loading and releasing the active ingredient by changes of pH.

Any process disclosed but not being part of the present invention can be used by the emulsification, such as sonication, use of a high potential disperser (such as ultra Turrax®) disperser, of a high pressure homogenizer, or of a high shear mechanical stirrer.

It is also disclosed but not being part of the present invention, a method for the release of at least one active compound, comprising the following steps:

- a step for adding at least one capsule but not being part of the present invention, encapsulating at least one hydrophilic or hydrophobic active compound as defined above, into an aqueous solution, and
- a step for adjusting the pH of said solution at a value of at least 6.5 or at a value of less than 4, and preferably of less than 3, in order to release the active compound into said aqueous solution.

The encapsulation and release processes not being part of the present invention may be used for health, cosmetic, food, personal, and home care applications where a compound must be first encapsulated and protected in an encapsulated phase. Targeted release by change of pH at the location to deliver the compound ensures higher activity of the compound. Such changes of pH can occur during endocytosis for cancer treatment, transfer from stomach to blood flow for food applications, absorption by the skin for cosmetic, wound healing and dermatology.

For the purposes of the current invention, any range given includes both the lower and the upper endpoints of the range.

## Brief description of drawings

Figure 1 concerns the variation of the hydrodynamic diameter of the capsules not being part of the present invention measured by DLS at 25°C obtained by sonication during 2 min with 50 wt% dodecane oil with an aqueous solution adjusted at pH 5.5 with citrate buffer containing 1 wt% diblock copolymers [$PEG_x$-*b*-P($Glu_n$-co-$Val_m$) with x = 44 or 114 and (n + m) = 5, 11 and 22 where m is around 30% of the total polypeptide DP, i.e. n + m] as function of the DP of the polypeptide block and prepared with 2 different PEG macroinitiators.

Figure 2 concerns the variation of the hydrodynamic diameter and the volume-average diameter determined by DLS and LD, respectively, of emulsion droplets obtained by sonicating during different time 50 wt.% n-dodecane with an aqueous solution of citrate buffer containing 1 wt.% of $PEG_{114}$-P($Glu_{16}$-co-$Val_5$) diblock copolymer. The black squares correspond to the volume-average diameter (in μm) and the black diamonds correspond to the hydrodynamic diameter (in nm).

Figure 3 concerns the variation of the hydrodynamic diameter determined by DLS of emulsion droplets obtained by sonicating during 10 min different amounts of n-dodecane oil with an aqueous solution of citrate buffer containing 5 wt % (black squares) or 10 wt % (black circles) of $PEG_{114}$-P($Glu_{16}$-co-$Val_5$) diblock copolymer.

Figure 4 concerns the variation of absorbance of the capsules not being part of the present invention based on n-dodecane prepared with $PEG_{44}$-P($Glu_{15}$-co-$Val_7$) (diamonds) and $PEG_{114}$-P($Glu_{46}$-co-$Val_{11}$) (triangles) redispersed in citrated buffer at pH 5.5 (open symbols) or in phosphate buffer at pH 7.4 (filled symbols).

Figure 5 concerns the variation of absorbance of the capsules not being part of the present invention based on octyl palmitate prepared with $PEG_{44}$-$PGlu_{58}$ (diamonds) and $PEG_{114}$-$PGlu_{151}$ (triangles) redispersed in citrated buffer at pH 5.5 (open symbols) or in phosphate buffer at pH 7.4 (filled symbols).

## Examples

### PREPARATION OF A COPOLYMER OF FORMULA (I)

Several diblock copolymers based on poly(ethylene glycol)-*b*-poly(glutamic acid-*co*-valine) [$PEG_x$P($Glu_n$-$Val_m$) or also written as $PEG_x$-*b*-P($Glu_n$-co-$Val_m$), with x = 16, 44 or 114 < n < 160 and 0 < m < 40] have been prepared.

### *Materials and Methods*

All chemicals were purchased from Sigma-Aldrich and were used as received. Methyl-and amine terminated polyethylene glycol (molecular weight of 2,000 g/mol and 5,000 g/mol) were purchased from RAPP polymer and were dried in toluene before use. γ-benzyl-*L*-glutamate N-carboxyanhydride (γ-BLG NCA) and *L*-valine N-carboxyanhydride (Val NCA) were purchased from Isochem. $^1$H NMR spectra were recorded on a Bruker AC 400 spectrometer. The molar masses and the polydispersities of poly(ethylene glycol)-*block*-poly(γ-benzyl-*L*-glutamate-co-*L*-valine) precursors were

determined by size exclusion chromatography (SEC) equipped with two PLgel mixed-columns (7.5 × 300 mm) and one PLgel 5 μm guard column (7.5 × 50 mm), two detectors have been used in this determination: a refractive index detector (Jasco 1530-RI), and an UV detector (Jasco 875-UV); dimethylformamide (DMF) has been used as eluent (0.8 mL/min) at 60°C in the presence of LiBr (1 g/L).

***Procedures***

*General synthesis method*

In order to obtain those copolymers, amine terminated PEG was used as initiator for the copolymerization of γ-benzyl-*L*-glutamate (BGL) and valine co-amino acids by ring opening copolymerisation (Scheme 1).

Well-defined diblock copolymers were obtained as judged by GPC analysis. $^1H$ NMR spectroscopy allowed the degree of polymerization of each co-amino acid to be calculated.

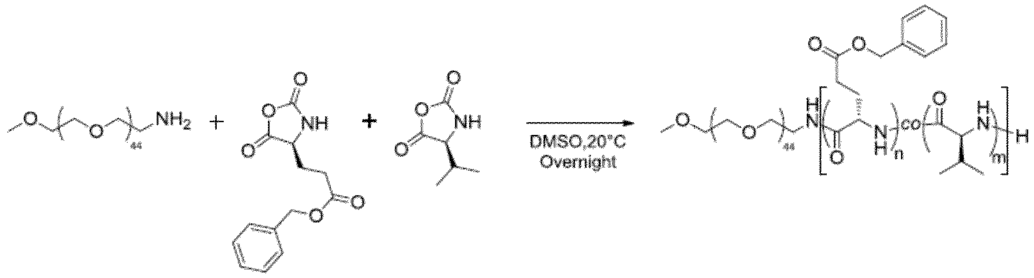


**Scheme 1. Schematic representation of the synthesis of $PEG_{44}$-P($BGL_n$-$Val_m$) by Ring Opening Polymerization.**

A second step consisting of the deprotection of the BLG units was carried out to obtain the corresponding $PEG_{44}$-P($Glu_n$-$Val_m$), as shown in Scheme 2.

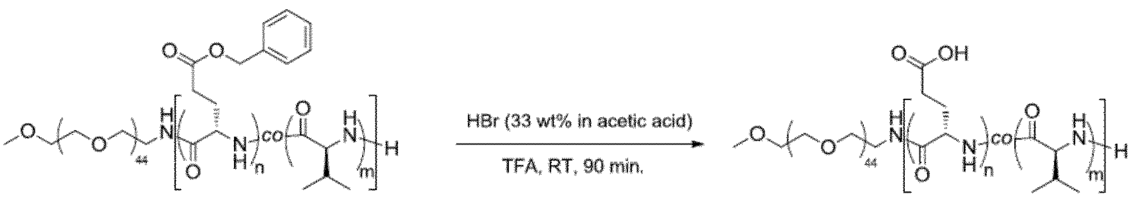


**Scheme 2. Deprotection step to obtain $PEG_{44}$-P($Glu_n$-$Val_m$)**

$PEG_{44}$-P($Glu_n$-$Val_m$) corresponds to a copolymer of formula (I) as defined above wherein x=44 and AA is valine.

The degree of deprotection, calculated by $^1H$ NMR, was greater than 90% for all copolymers.

Different degrees of polymerization for the PEG block and the polypeptide block were targeted to vary the interfacial activities of the diblock copolymers.

***General synthesis protocol to generate any block copolymers in the present family***

All chemicals were purchased from Sigma-Aldrich and were used as received. All N-carboxyanhydride NCA peptide monomers were purchased from Isochem. Various [monomers]/[PEG-macroinitiator] molar ratios were used to obtain copolymers with different hydrophilic weight fractions and molar masses.

For example, to prepare $PEG_{44}$-*b*-$PBLG_{25}$, PEG-$NH_2$ (Mn = 2,000 g/mol, 1 eq) was dissolved in dry toluene under nitrogen and degassed via 3 freeze-pump-thaw cycles and cryo-distilled to remove any water trace. Anhydrous solvent (preferably DMF or DMSO) was added to it. Then, γ-BLG NCA (25 eq to $NH_2$ function) was weighed in a glovebox under pure argon, introduced to a flame-dried Schlenk, and dissolved with 2 mL of anhydrous DMSO. The solution was stirred for 10 min and added with a nitrogen purged syringe to the PEG-$NH_2$ solution in anhydrous DMF or DMSO. The solution was stirred overnight to a few days at 5-20°C to reach full monomer conversion, precipitated in diethyl ether,

and dried under vacuum to afford a white powder. Same protocols were used to afford copolymers with different co-amino acids (combining benzyl glutamate and valine, but also phenyl alanine and others), the composition being controlled by the ratio of the N-carboxy Anhydride NCA monomers to PEG-$NH_2$ macroinitiator. Then, the deprotection of the benzyl groups of the benzyl glutamate can be performed using an acidic or basic deprotection method. Here, we preferred the acidic route that consists in dissolving the copolymers in HBr (33% in acetic acid solution) / TFA at room temperature for 90 minutes, to afford the final copolymers of formula (I) not being part of the present invention as defined above, which may also be represented by the formula $PEG_x$-P($Glu_n$-$AA_m$) (AA being as defined above).

***Detailed synthesis protocol to generate specific examples***

*Synthesis of polyethylene glycol)-b-poly(glutamic acid) $PEG_{44}$-P($Glu_n$): example of $PEG_{44}$-$PGlu_{58}$*

γ-BLG NCA (3.4 g, 14.5 mmol) was weighted in a glovebox under pure argon, introduced in a flame-dried Schlenk, and dissolved with 5 mL of anhydrous DMF. The solution was stirred for 10 min, and methyl- and amine-terminated polyethylene glycol (2KDa, 0.5 g, 0.25 mmol) was added with a nitrogen purged syringe from a solution in anhydrous DMF (1 g/mL, injected volume: 1 mL). The mixture was stirred for one day at 5°C (or at 25°C) precipitated in diethyl ether and dried under vacuum to afford a white powder. A polypeptide with a molecular mass of 12.702 $g.mol^{-1}$ (determined by $^1H$ NMR) corresponding to the targeted degree of polymerization of 58 was obtained. The corresponding copolymer was then dissolved in TFA (100 mg/ mL), and HBr solution (33 wt% in acetic acid) was added dropwise carefully. The reaction mixture was stirred for 90 minutes at room temperature and the polymer was precipitated twice in $Et_2O$ and dried under vacuum. It was then dissolved in DMSO, dialyzed against water (MWCO 1 KDa), and lyophilized to afford a white powder. Yield after purification: 87%

*Synthesis of poly(ethylene glycol)-b-poly(glutamic acid) $PEG_{114}$-P($Glu_n$): example of $PEG_{114}$-$PGlu_{151}$*

γ-BLG NCA (3.45 g, 15.1 mmol) was weighted in a glovebox under pure argon, introduced in a flame-dried Schlenk, and dissolved with 5 mL of anhydrous DMF. The solution was stirred for 10 min, and methyl- and amine-terminated polyethylene glycol (5KDa, 0.5 g, 0.1 mmol) was added with a nitrogen purged syringe from a solution in anhydrous DMF (1 g/mL, injected volume : 1 mL). The mixture was stirred for one day at 5°C (or at 25°C) precipitated in diethyl ether and dried under vacuum to afford a white powder. A polypeptide with a molecular mass of 33.069 $g.mol^{-1}$ (determined by $^1H$ NMR) corresponding to the targeted degree of polymerization of 151 was obtained.

**Typical $^1H$ NMR signals of PEG-*b*-PBLG (CDCl3/TFA)**: δ=7.85 (d, C(O)CH[(CH2)2C(O)OCH2Ph]N**H**; δ=7.30 (m, CH2**Ph**); δ=5.05 (m, C**H2**Ph); δ=4.55 (s, C(O)C**H**-[(CH2)2C(O)OCH2-Ph]NH); δ = 3.85 (s, O-C**H2**-C**H2** PEG) δ = 2.43 (t, C(O)CH[CH2C**H2**C(O)OCH2-Ph]NH); δ = 2.08, 1.92 (m, C(O)CH[(C**H2**CH2C-(O)OCH2-Ph]NH).

The corresponding copolymer was then dissolved in TFA (100 mg/ mL), and HBr solution (33 wt% in acetic acid) was added dropwise carefully. The reaction mixture was stirred for 90 minutes at room temperature and the polymer was precipitated twice in $Et_2O$ and dried under vacuum. It was then dissolved in DMSO, dialyzed against water (MWCO 1 KDa), and lyophilized to afford a white powder. Yield after purification: 83%

**Typical $^1H$ NMR signals of PEG-*b*-PGA (DMSO-d6/TFA):** δ=8.25 (s, C(O)CH-[(CH2)2C(O)OH]N**H**; δ=3.93 (m, C(O)CH-[(CH2)2C(O)OH]NH); δ = 3.44 (s, O-C**H2**-C**H2** PEG); δ=3.14 (s, C**H3**-(O-CH2-CH2)n PEG) δ=2.25 (m, C(O)CH[CH2C**H2**C(O)OH]-NH); δ = 1.90, (m, C(O)CH-[(C**H2**CH2C(O)OH]NH).

*Synthesis of poly(ethylene glycol)-b-poly(glutamic acid-co-valine) $PEG_x$-P($Glu_n$-$Val_m$): example of $PEG_{44}$-P($Glu_{15}$-$Val_7$)*

γ-BLG NCA (0.88 g, 3.75 mmol) Val NCA (0.25 g, 1.75 mmol) were weighted in a glovebox under argon, introduced in a flame-dried Schlenk, and dissolved with 5 mL of anhydrous DMSO. The solution was stirred for 10 min, and methyl- and amine-terminated polyethylene glycol (2KDa, 0.5 g, 0.25 mmol) was added with a nitrogen purged syringe from a solution in anhydrous DMSO (1 g/mL, injected volume : 1 mL). The mixture was stirred for one day at 25°C precipitated in diethyl ether and dried under vacuum to afford a white powder. A copolypeptide with a molecular mass of 3.978 $g.mol^{-1}$ (determined by $^1H$ NMR) corresponding to the total targeted degree of polymerization of 22 was obtained.

The corresponding copolymer was then dissolved in TFA (100 mg/ mL), and HBr solution (33 wt% in acetic acid) was added dropwise carefully. The reaction mixture was stirred for 90 minutes at room temperature and the polymer was precipitated twice in $Et_2O$ and dried under vacuum. It was then dissolved in DMSO, dialyzed against water (MWCO 1 KDa), and lyophilized to afford a white powder. Yield after purification: 82%

*Synthesis of poly(ethylene glycol)-b-poly(glutamic acid-co-valine) $PEG_x$-P($Glu_n$-$Val_m$): example of $PEG_{114}$-P($Glu_{51}$-$Val_6$)*

γ-BLG NCA (1.0 g, 4.6 mmol) and Val NCA (0.233 g, 1.1 mmol) were weighted in a glovebox under argon, introduced in a flame-dried Schlenk, and dissolved with 40 mL of anhydrous DMSO. The solution was stirred for 10 min, and methyl- and amine-terminated polyethylene glycol (5,000 $g.mol^{-1}$, 0.362 g, $7.24\times10^{-2}$ mmol) was added with a nitrogen purged syringe from a solution in anhydrous DMSO (1 $g.mL^{-1}$, injected volume : 0.36 mL). The mixture was stirred for two days at 25°C, dialyzed against water for 3 days (MWCO 1 KDa) and freeze-dried to afford a white powder. A copolypeptide with a total molecular mass of 11,763 $g.mol^{-1}$ corresponding to the total targeted degree of polymerization of 57 was obtained. Yield = 1.03 g (87 %)

**Typical $^1$H NMR signals of $PEO_{114}$-*b*-P($BLG_{51}$-*co*-$Val_6$) (DMSO-$d_6$/TFA):** δ=8.50-7.95 (m, C(O)CH-[(CH2)2C(O)OH]N**H** and C(O)-CH[CH(CH3)$_2$]-N**H**); δ=7.27 (m, CH2**Ph** BLG); δ=5.02 (m, C**H**$_2$Ph BLG); δ=3.94 (m, C(O)C**H**-[(CH2)2C(O)OCH2-Ph]NH and C(O)-C**H**[CH($CH_3$)$_2$]-NH); δ = 3.49 (s, O-C**H2**-C**H2** PEO); δ = 3.25 (s, C**H3**-O PEO); δ=2.73-1.75 (m, C(O)CH[CH2C**H2**C(O)OH]-NH and C(O)CH-[(C**H2**CH2C(O)OH]NH); δ=1.23 (m, C(O)-CH[C**H**(CH3)$_2$]-NH); δ=0.90 (m, C(O)-CH[CH(C**H3**)$_2$]-NH).

The corresponding copolymer (1 g, $5.95\times10^{-2}$ mmol, 3.03 mmol of BLG units) was then dissolved in TFA (100 $mg.mL^{-1}$), and HBr solution (33 wt% in acetic acid, 1.43 mL, 25 mmol ) was added dropwise carefully. The reaction mixture was stirred for 90 minutes at room temperature and the polymer was precipitated twice in $Et_2O$ and dried under vacuum. It was then dissolved in DMSO and water in presence of NaOH (pH = 9), dialyzed against water for 7 days (MWCO 1 KDa) and freeze-dried to afford a white powder. Yield: 700 mg (94 %) and total yield = 82 %

**Typical $^1$H NMR signals of $PEO_{114}$-*b*-P($Glu_{51}$-*co*-$Val_6$) (DMSO-$d_6$/TFA):** δ=8.50-7.95 (m, C(O)CH-[(CH2)2C(O)OH]N**H** and C(O)-CH[CH(CH3)$_2$]-N**H**); δ=3.94 (m, C(O)C**H**-[(CH2)2C(O)OCH2-Ph]NH and C(O)-C**H**[CH($CH_3$)$_2$]-NH); δ = 3.49 (s, O-C**H2**-C**H2** PEO); δ = 3.18 (s, **CH3**-O PEO); δ=2.70-1.80 (m, C(O)CH[CH2C**H2**C(O)OH]-NH and C(O)CH-[(C**H2**CH2C(O)OH]NH); δ=1.18 (m, C(O)-CH[C**H**(CH3)$_2$]-NH); δ=0.88 (m, C(O)-CH[CH(C**H3**)$_2$]-NH).

**Table 1: Summary of the different copolymers synthesized and their main characteristics**

| *Copolymer* (not being part of the present invention) | *PEG Mn ($g.mol^{-1}$)* | *n Glu* [a] | *m Val* [a] | *Mn polypeptide* [a] *($g.mol^{-1}$)* | *Mn Total copolymer ($g.mol^{-1}$)* | *Final Yield (%)* |
|---|---|---|---|---|---|---|
| $PEG_{44}$-$PGlu_{25}$ | 2,000 | 25 | 0 | 3,225 | 5,225 | 85 |
| $PEG_{44}$-$PGlu_{36}$ | 2,000 | 36 | 0 | 4,644 | 6,644 | 83 |
| $PEG_{44}$-$PGlu_{47}$ | 2,000 | 47 | 0 | 6,063 | 8,063 | 85 |
| $PEG_{44}$-$PGlu_{58}$ | 2,000 | 58 | 0 | 7,482 | 9,482 | 87 |
| $PEG_{44}$-$PGlu_{100}$ | 2,000 | 100 | 0 | 12,900 | 14,900 | 89 |
| $PEG_{114}$-$PGlu_{151}$ | 5,000 | 151 | 0 | 19,479 | 24,479 | 83 |
| $PEG_{114}$-$PGlu_{96}$ | 5,000 | 96 | 0 | 12,384 | 17,384 | not determined |
| $PEG_{44}$-P($Glu_{15}$-$Val_7$) | 2,000 | 15 | 7 | 2,628 | 4,628 | 82 |
| $PEG_{114}$-P($Glu_{16}$-$Val_5$) | 5,000 | 16 | 5 | 2,559 | 7,559 | 77 |
| $PEG_{114}$-P($Glu_{51}$-$Val_6$) | 5,000 | 51 | 6 | 7,173 | 12,173 | 82 |
| [a] *calculated from $^1$H NMR using PEG (s, O-C**H2**-C**H2**) resonance peak for calibration.* | | | | | | |

**PREPARATION OF CAPSULES**

The preparation processes of the capsules disclosed herein above and below do not form part of the present invention.

*General procedure*

A typical procedure to prepare the capsules not being part of the present invention consists in emulsifying equal volume of an aqueous solution containing from 1% to 20% by weight of the diblock copolymer and oil using a sonicator for 2 min. All the diblock copolymers tested were efficient stabilizers to prepare stable emulsions using different oils or

organic phase (dodecane, sunflower oil, olive oil, octyl palmitate and chloroform). The type of emulsions, i.e., oil-in-water or water-in-oil, was confirmed by the drop test technique.

The drop test technique consists in adding a drop of the final emulsion in an aqueous solution or the corresponding oil used for the emulsification. The phase which allows the droplets to redisperse indicates the dispersant phase, hence the other phase is the dispersed phase which is stabilized by the diblock copolymers. On the other hand, when the drop of the emulsion is added to the dispersed phase, no redispersion will be observed and the drop of emulsion will remain immiscible with the testing phase. Similarly, conductivity of the dispersant phase is usually used to confirm the droplet test. Oils usually have low conductivity ($\gamma$ < 1 $\mu S.m^{-1}$) compared to water which show good conductivity, typically > 100 $\mu S.m^{-1}$. Both tests confirmed oil-in-water emulsions were obtained in all cases when dodecane, sunflower and olive oil were used and the water phase containing the diblock copolymer not being part of the present invention was adjusted at pH 5.5. Water droplets were redispersed when chloroform was used as the immiscible phase.

The preparation processes of the emulsions disclosed herein above and below do not form part of the present invention.

***General protocol for oil-in-water emulsion*** not being part of the present invention

A typical procedure for 2 g of oil-in-water emulsion at 50% oil to emulsify was as follows, first 1 g of an aqueous solution containing the diblock copolymer at concentration between 1 and 10 wt% adjusted at pH 5.5 with HCl or with citrate buffer was added into a 5 mL vial. Then, 1 g of the hydrophobic phase (dodecane oil, sunflower oil, olive oil, octyl palmitate, jojoba oil, isononyl isononanoate, squalene, isohexadecane, Caprylic/capric triglyceride, Isodecyl isononanoate) was added to the vial.

This oil/water mixture was then homogenized for 2 minutes (other sonication times have been employed, 4, 6 and 8 minutes) using a sonicator UP400S (HIELSCHER). It is noteworthy that the mixture was placed an ice bath to limit the heating from the highly energetic sonication process.

Table showing the different quantity tested for a final emulsion of 2 g

| Emulsion concentration (wt%) | Concentration of emulsifier based on the amount of oily phase used (wt%) | Amount of aqueous phase (g) | Amount of emulsifier (g) | Amount of oily phase (g) |
|---|---|---|---|---|
| 50 | 1 | 0.99 | 0.01 | 1 |
| 50 | 5 | 0.95 | 0.05 | 1 |
| 50 | 10 | 0.9 | 0.1 | 1 |
| 20 | 1 | 0.996 | 0.004 | 0.4 |
| 20 | 5 | 0.98 | 0.02 | 0.4 |
| 20 | 10 | 0.96 | 0.04 | 0.4 |
| 10 | 1 | 0.998 | 0.002 | 0.2 |
| 10 | 5 | 0.99 | 0.01 | 0.2 |
| 10 | 10 | 0.98 | 0.02 | 0.2 |

The amount of copolymer of formula (I) and (II) to produce water-in-oil emulsion not being part of the present invention could be varied between 0.1wt% and 20 wt% based on the total weight of the formulation and preferably between 0.2 wt% and 10 wt% and more preferably between 0.5 wt% and 5 wt%.

***General protocol for water-in-oil emulsion*** not being part of the present invention

A typical procedure for 2 g of water-in-oil emulsion not being part of the present invention at 50 wt% aqueous phase to emulsify was as follows, first 1 g of chloroform or dichloromethane containing the diblock copolymer at concentration at 1 wt% was added into a 5 mL vial. Then, 1 g of the aqueous phase adjusted at pH 5.5 with HCl or with citrate buffer was added to the vial.

This organic solvent/water mixture was then homogenized for 2 minutes using a sonicator UP400S (HIELSCHER). It is noteworthy that the mixture was placed an ice bath to limit the heating from the highly energetic sonication process.

Similar water-in-oil emulsion not being part of the present invention was obtained when the diblock copolymer

was dissolved in the aqueous phase instead of the chloroform or dichloromethane phase.

Control experiments using PEG homopolymer confirmed that PEG does not show any interfacial activity and cannot be used as macroemulsifier. On the other hand, PGA homopolymer has shown interfacial activity (J. Am. Chem. Soc. 2006, 128, 6540-6541) and was able to form capsules. However, such capsules appeared to be unstable in the presence of salts and confirmed that the diblock architecture of the macroemulsifier is crucial to obtain stable nanocapsules.

***Protocol for the control experiment using PEG homopolymer as emulsifier for oil-in-water emulsion***

A typical procedure for oil-in-water emulsion, first 1 g of an aqueous solution containing $PEG_{114}$ homopolymer with a molecular weight of 5,000 g $mol^{-1}$ or $PEG_{44}$ homopolymer diacrylate (PEGDA) with a molecular weight of 2,000 g $mol^{-1}$ at concentration between 10 wt% was added into a 5 mL vial. Then, 1 g of the hydrophobic phase (dodecane oil, sunflower oil, olive oil, octyl palmitate or isohexadecane) was added to the vial.

This oil/water mixture was then homogenized during 10 minutes using a sonicator UP400S (HIELSCHER). It is noteworthy that the mixture was placed an ice bath to limit the heating from the highly energetic sonication process.

Phase separation occurred in a matter of minutes after 10 min sonication of an oil/water mixture based on 1 g of aqueous solution containing 10 wt% PEGDA and 1 g of dodecane. Changing the PEGDA for PEG homopolymer did not achieve the production of stable emulsion with dodecane. Similar results were obtained with different oils tried, such as sunflower oil, olive oil, octyl palmitate or isohexadecane for both PEGDA and PEG. These results confirmed that PEG alone, without the polypeptide block of formula (I) and (II), is not able to stabilize emulsions.

***General protocol for cross-linked capsules*** not being part of the present invention ***with diamine compounds***

The same protocol as for the oil-in-water not being part of the present invention or water-in-oil not being part of the present invention at 50 wt% using 1wt% copolymer was followed except that different amounts of ethylene diamine or bis(hexamethylene) triamine were added in the phase which did not contain the copolymer such as the ratio of primary amine from those compounds and the number of carboxylic acid from the glutamic acid residues of the diblock copolymer was $NH_2$:COOH: 1:1, 1:2 and 1:4. When the diamine compound was placed in the water phase, 10 times excess of ethyl(dimethylaminopropyl) carbodiimide (EDC) based on the total amount of carboxylic acid unit from the glutamic acid units of the diblock copolymer were added. Similarly, 10 times of dicyclohexylcarbodiimide (DCC) based on the amount of carboxylic acid dissolved in the aqueous phase to produce the emulsion were added to the water-immiscible phase to cross-link the copolymer.

It is important to note that inter-crosslinking between different droplets was observed with the higher amount of diamine cross-linker added to the continuous phase (water for oil-in-water system and water-immiscible phase for water-in-oil system) was carried out.

**Size of the capsules not being part of the present invention.**

The size of the capsules not being part of the present invention could be decreased by increasing the degree of polymerization of both block PEG and polypeptides as shown in Figure 1. The hydrodynamic diameter of the capsule was measured by Dynamic Light Scattering in the experimental conditions explained above.

This result combined with the lack of solubility of the diblock copolymers in the oil phase gives an indication of the adsorption of the macroemulsifier at the oil/water interface. However, smaller capsules not being part of the present invention were obtained with longer polypeptides block. Therefore, the mechanism of stabilization could be assumed as the adsorption of the hydrophobic polypeptide at the oil/water interface. In that case, longer polypeptide will cover a greater area at the oil/water interface, resulting in smaller capsules not being part of the present invention. It is important to mention that the steric hindrance of the PEG might also affect the stabilization mechanism. Its effect should also go in the same way as longer PEG might require more space. This is in good agreement with the results obtained as sufficiently long PGlu is actually required to obtain stable emulsion.

In more detail, for PEG-PGlu diblock copolymers, a minimum degree of polymerization of the Glu amino acids was required to obtain stable emulsions, i.e. DP > 20, 36, and 50 for PEG with DP 16, 44 and 114, respectively. This will be shown below when $PEG_{44}$-$PGlu_{36}$ was used as emulsifier and that stable emulsions could not be achieved with any of the oils used at low pH (around pH 5.5).

Interestingly, the diameters of the capsules not being part of the present invention could be even lowered by increasing the amount of copolymers and time of sonication. It is interesting to see that the diameters of the capsules not being part of the present invention could be decreased by a factor of 3 only by applying a strong sonication for at least 10 min (Figure 2). The diameter of the capsule was measured by Dynamic Light Scattering in the experimental conditions explained above and the trend of smaller size at higher sonication time was also confirmed by laser diffraction.

Similarly, the diameter of the capsules not being part of the present invention could be reduced by increasing the amount of diblock copolymer and decreasing the amount of the dispersed phase (Figure 3).

***Protocol for the production of oil-in-water emulsion* not being part of the present invention *with different PEG-PGlu.***

A typical procedure for 2 g of oil-in-water emulsion at 50% dodecane oil to emulsify was as follows, first 1 g of an aqueous solution of buffer citrate at pH 5.5 containing a 1 wt% of diblock copolymer, namely [$PEG_x$-P($Glu_n$-$Val_m$) with x = 44 or 114 and (n + m) = 5, 11 and 22 where m is around 30 % of the total polypeptide DP, i.e. n + m]. Then, 1 g of dodecane oil was added to the vial.

The mixture was sonicated during 2 min using a sonicator UP400S (HIELSCHER) at full power with no pulse time.

***Protocol for the production of oil-in-water emulsion* not being part of the present invention *at different sonication time***

A typical procedure for 2 g of oil-in-water emulsion at 50 % dodecane oil to emulsify was as follow, first 1 g of an aqueous solution of buffer citrate at pH 5.5 containing a 1 wt% of $PEG_{114}$-P($Glu_{16}$-$Val_5$) diblock copolymer. Then, 1 g of dodecane oil was added to the vial. This oil/water mixture was then homogenized for 2 minutes (other sonication times have been employed, 4, 6 and 8 minutes) using a sonicator UP400S (HIELSCHER). It is noteworthy that the mixture was placed an ice bath to limit the heating from the highly energetic sonication process.

**STABILITY**

All capsules not being part of the present invention based on dodecane, olive oil, sunflower oil, octyl palmitate and isohexadecane and stabilized with $PEG_x$-P($Glu_n$-$Val_m$) diblock copolymers with x = 44 or 116 and 5 < n < 50 and 1 < m < 11 were stable for months at pH 5.5. In the case of $PEG_x$-$PGlu_m$, the absence of Valine coamino acids was clearly decreasing the interfacial activity of the diblock copolymer and the formation of stable nanocapsules was depending on the length of both the PEG block and the polypeptide block. For example, capsules could not be prepared with a diblock copolymer containing a short PEG block, i.e. x = 17, and any of the oils mentioned above. With intermediate length PEG block, it was found that capsules based on $PEG_{44}$-$PGlu_{36}$ could not be produced with any of the oils, except octyl palmitate. On the other hand, stable capsules were prepared with $PEG_{44}$-$PGlu_{47}$ and $PEG_{44}$-$PGlu_{58}$ with n-dodecane, sunflower oil and olive oil. Interestingly, when $PEG_{117}$-$PGlu_m$ diblock copolymers, stable capsules were achieved with shorter PGlu, i.e. m ≥ 20. On the other hand, stability issues were found when octyl palmitate was emulsified. This could be attributed to the different polarity of this oil. With octyl palmitate, stable capsules could only be obtained with $PEG_{117}$-$PGlu_n$ with n > 50.

***Protocol to study the stability of the emulsions* not being part of the present invention**

First, when the emulsions were prepared as previously described, visual inspection was used to determine the short term stability as phase separation was observed. To do so, the emulsion was left resting in normal conditions at room temperature. The appearance of oil droplets at the surface of the dispersion was considered as loss of stability and the emulsions that did not show phase separation after a week were considered for further long term stability studies carried out by dynamic light scattering. Every week, 2 μl of the emulsion was redispersed in 1 mL of deionized water and the hydrodynamic diameter was measured by DLS. If the hydrodynamic diameter remained stable, the emulsion was considered as stable. On the contrary if an increase of the hydrodynamic diameter was observed the emulsion was considered as unstable.

More interestingly, $PEG_x$-P($Glu_n$-$Val_m$) diblock copolymers appeared to show different stability depending on the pH of emulsification.

Stable nanocapsules based on olive oil were obtained with $PEG_{44}$-$PGlu_{47}$ and $PEG_{44}$-$PGlu_{58}$ diblock copolymers at pH 5.5. However, the same emulsions prepared at pH > 7.4 were not stable and demulsification occurred within a month. This result indicates that the interfacial activity of the diblock copolymer depends on the pH. Such behaviour was attributed to the change of character of the PGlu block. Obviously, PEG block is water soluble independently of pH, but PGlu block is showing increasing hydrophobicity at lowering the pH, due to the protonation of the carboxylic acid residues of the amino acids units and hydrophilic at pH > pKa with the presence of carboxylate anions. Thus, at low pH, the polypeptide block is sufficiently hydrophobic to adsorb at the oil/water interface. On the other hand, when PGlu is hydrophilic the lack of affinity with the hydrophobic interface resulted in the absence of interfacial activity for the macroemulsifier and stable emulsions could not be achieved. It is very important to mention that at very low pH (< 3), demulsification also occurs due to the rearrangements of the PGlu block in α-helix, as previously described (J. Am. Chem.

Soc. 2006, 728, 6540-6541).

Therefore, PEG-PGlu copolymers exhibit a window of stability for the resulting emulsion when sufficient Glu amino acids units are protonated and hydrophobic to ensure adsorption of the block at the oil/water interface but not too many to avoid the inherent rearrangement of the polypeptide block in its natural configuration.

More interestingly, the interfacial activity of the diblock copolymer PEG-PGlu could be changed *in situ* which resulted in the desorption or detachment of the diblock copolymers from the oil-water interface, leading to the release of the core of the capsule. For example, stable capsules not being part of the present invention based on n-dodecane prepared at pH 4.5 and stabilized by $PEG_{44}$-$PGlu_{58}$ could be demulsified by adjusting the pH above pH 7.4 and gentle shacking of the dispersion of emulsion droplets. In addition, new capsules not being part of the present invention could be produced by readjusting the pH of the aqueous phase down to pH between 4.5 and 5.5. After 2 min sonication, stable emulsion was again obtained. This change *in situ* of the interfacial activity of the diblock copolymer proved that the macroemulsifier is pH responsive.

***pH-dependence of the nanocapsules*** not being part of the present invention

To assess the pH-dependence of the capsules, the emulsification process was carried out at different pHs, i.e. 5.5 (like previously described), 7.4 and 2. To do so, the aqueous solution used to dissolve the copolymer was adjusted with concentrated NaOH or HCl for pH 7.4 and 2, respectively. After preparing the biphasic mixture as explained above, sonication was carried out between 2 and 10 min. Visual inspection to check the demulsification was used to verify the stability of those emulsions at pH 7.4 and pH 2. In all the cases, capsules previously stable at pH 5.5 proved to be unstable as demulsification was observed when the emulsion was carried out at pH 7.4 and 2.

***pH-responsiveness of the capsules*** not being part of the present invention

In order to demonstrate the effect of the pH on the stability of the emulsions, capsules which already showed high stability at pH 5.5 were adjusted at pH 7.4 by addition of concentrated NaOH or by redispersing the nanocapsules at pH 7.4 in phosphate buffer. First visual inspection was used to verify the capsules stability. When the capsules were pH-responsive the milky solution became clearly transparent.

Within a couple of hours, the emulsions adjusted at pH 7.5 became transparent whereas the control experiments maintained at pH 5.5 remained milky.

Using the change in absorbance of the emulsions after pH changes, UV-spectroscopy (Shimadzu UV-2401 UV/Vis spectrophotometer) was used to monitor the demulsification after changing the pH. To do so, 5 $\mu$L of the original dispersion was redispersed in phosphate buffer at pH 7.4. The absorbance of the capsules dispersion at 500 nm was monitored with time (Figure 4).

From Figure 4, it is clear that when the capsules prepared with diblock copolymers with a block polypeptide contain Val and Glu coamino acids demulsification occurred at pH 7.4 with significant decrease in the dispersion absorbance. However, it could be seen that for both capsules the absorbance first increases and then decreases dramatically. This increase was attributed to the first destabilization of the capsules which coalesced to larger particles with higher diffraction, hence higher absorbance at first. After this coalescence step, the capsules were completely destroyed resulting in a transparent solution. On the other hand, when the same capsules were placed in buffer citrate at pH 5.5, the absorbance remained constant demonstrating the stability of the colloidal system. In the case of capsules prepared with $PEG_x$-$PGlu_n$ or $PGlu_n$-$A_1$-$PEG_x$ diblock copolymers, similar trend was observed except that the pH-responsiveness appeared to be slower with full demulsification occurring after 15 hrs.

Visual inspection of the DLS cuvette confirmed that demulsification occurred with the appearance of oily stains at the surface of the aqueous dispersion.

Separately, chloroform was also used as immiscible phase.

In this case, different results were obtained. A phase inversion was observed depending on the length of the PEG block. For example with a relatively short PEG block (DP =17), chloroform-in-water emulsions were formed. However, with longer PEG block (44 and 114), water-in-chloroform emulsions were obtained. It appears that the PEG which is soluble in both, water, and chloroform, prefers to be in the organic phase when the chains are sufficiently long.

This has only been observed for chloroform as immiscible phase because PEG was not soluble in the other oils.

**CROSSLINKING**

To improve stability and to produce a new type of capsules not being part of the present invention, it was decided to cross-link in situ the Glu amino acids residues adsorbed at the oil/water interface.

The adsorbed diblock copolymers were cross-linked via amidation of the Glu units using a diamine compound like ethylene diamine or bis(hexamethylene) triamine. Due to the lack of solubility of the diamine cross-linker in dodecane,

the cross-linking reaction was carried out through the aqueous phase. First, the carboxylic acids of the diblock adsorbed at the oil/water interface were activated using EDC. Then, the diamine cross-linker was added slowly to the diluted dispersion of n-dodecane in water capsules.

First the amount of diamine cross-linker was added to react with all the Glu units present in the water dispersion assuming that all the copolymer was adsorbed at the interface. Optical microscope studies showed some deformation of the original droplets that might indicate successful cross-linking of the adsorbed copolymer. Islets of droplets were also observed which indicates cross-linking neighboring droplets. Inter cross-linking of droplets was expected due to the high amount of cross-linker added.

Lower amount of diamine cross-linker was used to minimize droplet inter-cross-linking. Deformations of the droplet after cross-linking were still observed but fewer aggregates appeared to be present. However, a closer look at the optical images indicates an increase of the diameter for the droplets after the cross-linking reaction. This result confirms that the inter droplet cross-linking also occurred at this concentration of cross-linker.

Then the diblock copolymers adsorbed at the oil/water interface were cross-linked using only 25 mol% of the available Glu units (assuming that all diblock copolymer is adsorbed at the oil/water interface). Optical microscope pictures confirmed that intercross-linking of oil droplets was minimized while the droplet diameter remained similar to the original one, especially for $PEG_{44}$-$PGlu_{58}$ stabilized droplet at pH 2.6. It is worth noting that some of the droplets did not exhibit spherical shape which might be due to a mechanical stress generated by the cross-linking reaction.

$PEG_x$-$PGlu_n$ block copolymer were also cross-linked using bis(hexamethylene) triamine in order to incorporate a secondary amine which exhibits a pKa around pH 8. Similarly as the results obtained with ethylene diamine, large droplets appeared to have lost their spherical shape. On the other hand, small droplets remain spherical and their diameter remained unchanged.

In summary, the capsules prepared from a water-in-chloroform emulsion template exist in different state upon the cross-linker used and the pH of the dispersion. When bis(hexylmethylene) triamine was used as cross-linker, the capsules were negatively charged with a swollen membrane due to the high solubility of the polypeptide block. Deswelling of the membrane was observed at decreasing the dispersion pH due to the protonation of the carboxylic acid of the GA units. At pH < 4, smaller capsules were obtained with a cationic charge but the presence of the protonated secondary amine might give enough hydrophilicity to the membrane to be slightly swollen and not completely impermeable. Similarly, the capsules cross-linked with ethylene diamine exhibited a swollen and anionic membrane at alkaline pH. The membrane thickness was shrinking when the pH was decreased due to the protonation of the Glu amino acid units and the loss of electrostatic repulsion. At pH < 4, the membrane of the capsule is completely closed due to the hydrophobic character of the polypeptide block. Moreover, the shrinking observed at pH 3 might ensure the formation of a protective barrier to encapsulate hydrophilic compound.

Example I: Oil-in-glycerol emulsion for the encapsulation of *Curcuma Longa* Root Extract using $PEG_{114}$-P($Glu_{46}$-*co*-$Val_{11}$)

| Formulation | | |
|---|---|---|
| | Wt% | Weight (g) |
| $PEG_{114}$-P($Glu_{46}$-*co*-$Val_{11}$) | 1 | 0.06 |
| Curcuma Longa Root Extract | 7.5 | 0.45 |
| Octyl Palmitate | 2.5 | 0.15 |
| Glycerol | 89 | 5.34 |

*Emulsification process*

Polypeptide based block copolymer ($PEG_{114}$-P($Glu_{46}$-*co*-$Val_{11}$)) was first dissolved in glycerol at 80°C for 1h and ambient temperature under magnetic stirring then (18h, 250 rpm). After that, a pre-emulsion was formed by the addition of octyl palmitate and Curcuma Longa Root Extract mixture (RT, 250 rpm, 10') to the glycerol phase. The mixture was agitated at room temperature for 30 minutes (250 rpm). Quantities of each component are shown in the formulation above.

Emulsification consisted in the sonication of the mixture using a sonicator UP400S (Hielscher) at 100 % amplitude and 1.0 cycles for 10 min (400 Hz) stirred at 500 rpm.

*Emulsion characterization*

Appearance: the appearance of the final nanocapsules was analysed visually. Clear viscous good quality emulsion (without sedimentation) was achieved.

Particle size: mean particle size intensity and polydispersity index were measured by Zetasizer Nano ZS (Malvern). Samples were prepared by diluting the emulsion with DDI water till a concentration close to 1 wt%. Measurements were carried out in triplicate at 25°C.
Zetasizer Nano Zs: Particle size (nm) = 125 nm; PDI=0.25

Example II: Oil-in-glycerol emulsion for the encapsulation of Ceramide NG using $PEG_{114}$-P($Glu_{46}$-*co*-$Val_{11}$)

| Formulation | | |
|---|---|---|
| | Wt% | Weight (g) |
| $PEG_{114}$-P($Glu_{46}$-*co*-$Val_{11}$) | 1 | 0.15 |
| Ceramide NG | 1 | 0.15 |
| Sodium Dilauramidoglutamide Lysine (Pellicer L-30) | 1 | 0.15 |
| Octyldodecanol | 8 | 1.2 |
| Glycerol | 89 | 13.35 |

*Emulsification process*

Polypeptide based block copolymer ($PEG_{114}$-P($Glu_{46}$-*co*-$Val_{11}$)) was dissolved in glycerol first at 80°C for 1 hour and then, at ambient temperature under magnetic stirring (18h, 250 rpm). The mixture was further adjusted to 95°C for 10 minutes. On the other hand, Ceramide NG, Sodium Dilauramidoglutamide Lysine and Octyldodecanol were mixed for 10 minutes at 95°C (250 rpm). After that, a pre-emulsion was formed by mixing the octyldodecanol phase to the glycerol phase at 95°C for 20 minutes (250rpm). Quantities of each component are shown in the formulation above.

Emulsification consisted in the sonication of the mixture using a sonicator UP400S (Hielscher) at 100 % amplitude and 1.0 cycles for 10 min (400 Hz) stirred at 500 rpm and at 60°C (desired temperature reached using an oil bath).

*Emulsion characterization*

Appearance: the appearance of the final nanocapsules was analysed visually. Milky viscous good quality emulsion (without sedimentation) was achieved.

Particle size: mean particle size intensity and polydispersity index were measured by Zetasizer Nano ZS (Malvern). Samples were prepared by diluting the emulsion with DDI water till a concentration close to 1 wt%. Measurements were carried out in triplicate at 25°C.
Zetasizer Nano Zs: Particle size (nm) = 150 nm; PDI=0.05

Example III: Oil-in-glycerol emulsion for the encapsulation of Retinyl palmitate encapsulated using $PEG_{114}$-P($Glu_{46}$-*co*-$Val_{11}$)

| Formulation | | |
|---|---|---|
| | Wt% | Weight (g) |
| $PEG_{114}$-P($Glu_{46}$-*co*-$Val_{11}$) | 1 | 0.06 |
| Retinyl palmitate | 7.5 | 0.45 |
| Octyl palmitate | 2.5 | 0.15 |
| Glycerol | 89 | 5.34 |

*Emulsification process*

Polypeptide based block copolymer ($PEG_{114}$-P($Glu_{46}$-*co*-$Val_{11}$)) was dissolved in glycerol at 80°C for 1h hour and kept at ambient temperature under magnetic stirring (18h, 250 rpm). After that, a pre-emulsion was formed by the addition of octyl palmitate and retinyl palmitate mixture (RT, 250 rpm, 10') to the glycerol phase. The mixture was agitated (RT, 250 rpm, 30'). Quantities of each component are shown in the formulation above. Emulsification consisted in the sonication of the mixture using a sonicator UP400S (Hielscher) at 100 % amplitude and 1.0 cycles for 10 min (400 Hz) stirred at 500 rpm.

*Emulsion characterization*

Appearance: the appearance of the final nanocapsules was analysed visually. Clear/yellowish viscous good quality emulsion (without sedimentation) was achieved. Particle size: mean particle size intensity and polydispersity index were measured by Zetasizer Nano ZS (Malvern). Samples were prepared by diluting the emulsion with DDI water till a concentration close to 1 wt%. Measurements were carried out in triplicate at 25°C.
Zetasizer Nano Zs: Particle size (nm) = 135 nm; PDI=0.09

Example IV: Oil-in-glycerol emulsion for the encapsulation of Curcuma Longa Root Extract using $PEG_{114}$-$PGlu_{151}$

| Formulation | | |
|---|---|---|
| | Wt% | Weight (g) |
| $PEG_{114}$-$PGlu_{151}$ | 1 | 0.04 |
| Curcuma Longa Root Extract | 7.5 | 0.3 |
| Octyl Palmitate | 2.5 | 0.1 |
| Glycerol | 89 | 3.56 |

*Emulsification process*

Polypeptide based block copolymer ($PEG_{114}$-$PGlu_{151}$)) was first dissolved in glycerol at 80°C for 1h and ambient temperature under magnetic stirring then (18h, 250 rpm). After that, a pre-emulsion was formed by the addition of octyl palmitate and Curcuma Longa Root Extract mixture (RT, 250 rpm, 10') to the glycerol phase. The mixture was agitated at room temperature for 30 minutes (250 rpm). Quantities of each component are shown in the formulation above.

Emulsification consisted in the sonication of the mixture using a sonicator UP400S (Hielscher) at 100 % amplitude and 1.0 cycles for 10 min (400 Hz) stirred at 500 rpm.

*Emulsion characterization*

Appearance: the appearance of the final nanocapsules was analysed visually. Clear viscous good quality emulsion (without sedimentation) was achieved.

Particle size: mean particle size intensity and polydispersity index were measured by Zetasizer Nano ZS (Malvern). Samples were prepared by diluting the emulsion with DDI water till a concentration close to 1 wt%. Measurements were carried out in triplicate at 25°C.
Zetasizer Nano Zs: Particle size (nm) = 255 nm; PDI=0.20

Example V: Oil-in-glycerol emulsion for the encapsulation of Ceramide NG using $PEG_{114}$-$PGlu_{151}$

| Formulation | | |
|---|---|---|
| | Wt% | Weight (g) |
| $PEG_{114}$-$PGlu_{151}$ | 1 | 0.04 |

(continued)

| Formulation | | |
|---|---|---|
| | Wt% | Weight (g) |
| Ceramide NG | 1 | 0.04 |
| Sodium Dilauramidoglutamide Lysine (Pellicer L-30) | 1 | 0.04 |
| Octyldodecanol | 8 | 0.32 |
| Glycerol | 89 | 3.56 |

*Emulsification process*

Polypeptide based block copolymer ($PEG_{114}$-$PGlu_{151}$) was dissolved in glycerol at 80°C for 1h first and ambient temperature under magnetic stirring then (18h, 250 rpm). The mixture was further adjusted to 95°C for 10 minutes. On the other hand, Ceramide NG, Sodium Dilauramidoglutamide Lysine and Octyldodecanol were mixed for 10 minutes at 95°C (250 rpm). After that, a pre-emulsion was formed by mixing the octyldodecanol phase to the glycerol phase. The mixture was agitated (95°C, 250 rpm, 20'). Quantities of each component are shown in the formulation above.

Emulsification consisted in the sonication of the mixture using a sonicator UP400S (Hielscher) at 100 % amplitude and 1.0 cycles for 10 min (400 Hz) stirred at 500 rpm and at 60°C (desired temperature reached using an oil bath).

*Emulsion characterization*

Appearance: the appearance of the final nanocapsules was analysed visually. Milky viscous good quality emulsion (without sedimentation) was achieved.

Particle size: mean particle size intensity and polydispersity index were measured by Zetasizer Nano ZS (Malvern). Samples were prepared by diluting the emulsion with DDI water till a concentration close to 1 wt%. Measurements were carried out in triplicate at 25°C.

Zetasizer Nano Zs: Particle size (nm) = 160 nm; PDI=0.04

Example VI: Oil-in-glycerol emulsion for the encapsulation of Retinyl palmitate using $PEG_{114}$-$PGlu_{151}$

| Formulation | | |
|---|---|---|
| | Wt% | Weight (g) |
| $PEG_{114}$-$PGlu_{151}$ | 1 | 0.150 |
| Retinyl palmitate | 7.5 | 1.125 |
| Octyl palmitate | 2.5 | 0.375 |
| Glycerol | 89 | 13.35 |

*Emulsification process*

Polypeptide based block copolymer ($PEG_{114}$-$PGlu_{151}$) was dissolved in glycerol at 80°C for 1h and at ambient temperature for 18 h under magnetic stirring (250 rpm). After that, a pre-emulsion was formed by the addition of octyl palmitate and retinyl palmitate mixture (RT, 250 rpm, 10') to the glycerol phase. The mixture was agitated (RT, 250 rpm, 30'). Quantities of each component are shown in the formulation above.

Emulsification consisted in the sonication of the mixture using a sonicator UP400S (Hielscher) at 100 % amplitude and 1.0 cycles for 10 min (400 Hz) stirred at 500 rpm.

*Emulsion characterization*

Appearance: the appearance of the final nanocapsules was analysed visually. Clear/yellowish viscous good quality emulsion (without sedimentation) was achieved. Particle size: mean particle size intensity and polydispersity

index were measured by Zetasizer Nano ZS (Malvern). Samples were prepared by diluting the emulsion with DDI water till a concentration close to 1 wt%. Measurements were carried out in triplicate at 25°C.
Zetasizer Nano Zs: Particle size (nm) = 260 nm; PDI=0.2

## SYNTHESIS OF A COPOLYMER OF FORMULA (II) WITH A LINKER

In the above examples, the used copolymers may be represented on the basis of formula (II) as defined above, in particular with the following nomenclature: poly(glutamic acid-co-valine)-*b*-βA-PVGLIG-*b*-poly(ethylene glycol) [P($Glu_n$-co-$Val_m$)-*b*-βA-PVGLIG-*b*-$PEG_{114}$.

With the aim of obtaining copolymers of formula (II) not being part of the present invention as defined above, an enzyme-specific cleavable peptide sequence, namely βAla-ProValGlyLeuIleGly (noted βAla-PVGLIG in 1 letter amino acid code), was used as an enzyme-cleavable linker in between the polymer blocks. More specifically, the following copolymers were synthesized: poly(glutamic acid-*co*-valine)-*b*-βA-PVGLIG-*b*-poly(ethylene glycol) [P($Glu_n$-co-$Val_m$)-*b*-βA-PVGLIG-*b*-$PEG_{114}$ with $61 \leq n \leq 170$ and $0 \leq m \leq 16$]. When m=0, the block copolymer is noted $PGlu_n$-*b*-βA-PVGLIG-*b*-$PEG_{114}$. The synthesis is performed in four main steps.

Step 1: Firstly, the Fmoc-protected peptide sequence Fmoc-βA-PVGLIG-OH peptide (βA stands for beta-alanine and was introduced as a short linker) was dissolved in dry DCM (20 mg, 0.0235 mmol) and stirred for 10 min. Then, methyl- and amine-terminated polyethylene oxide (5,000 $g.mol^{-1}$, 98 mg, 0.0196 mmol) was added with a nitrogen-purged syringe from a solution in anhydrous DCM (0.1 mg/mL, injected volume: 800 μL), followed by the addition of EDC.Cl (7.5 mg, 0.0039 mmol) and DMAP (0.48 mg, 0.39 μmol). The solution was stirred for 24 hours at 20°C, purified by extraction using DCM and water, followed by precipitation in $Et_2O$, and dried under high vacuum.

Step 2: Removal of the N-terminal Fmoc group of the peptide was achieved by dissolving Fmoc-βA-PVGLIG-*b*-poly(ethylene oxide)$_{114}$ in DMF (15 mg/mL) and adding a solution of piperidine dropwise to a final ratio Piperidin/DMF (1:4). The reaction mixture was stirred for 45 min at room temperature. The conjugate H-βA-PVGLIG-*b*-poly(ethylene oxide)$_{114}$ was purified by dialysis against MilliQ water (MWCO 1 kDa), followed by freeze-drying. Step 3: For the next step, NCA-BLG (0.28 g, 1.0 mmol) was weighed in a glovebox under pure argon, introduced into a flame-dried Schlenk, and dissolved with 2 mL anhydrous DMF. The solution was stirred for 10 min, and H-βA-PVGLIG-*b*-poly(ethylene oxide)$_{114}$ (60 mg, 0.01 mmol) was added with a nitrogen-purged syringe from a solution in anhydrous DMF. The solution was stirred overnight at 25°C, precipitated in $Et_2O$, washed 3 times, and dried under vacuum.

Step 4: Finally, P(BLG)-*b*-βA-PVGLIG-*b*-$PEG_{114}$ was dissolved in TFA (50 mg/mL), followed by the addition of a HBr solution (33 wt% in acetic acid, 257 μL, 1.5 mmol). The reaction mixture was stirred for 3 hours at room temperature and the diblock copolymer $PGlu_x$-*b*-βA-PVGLIG-*b*-$PEG_{114}$ was precipitated twice in $Et_2O$ and dried under vacuum. It was then dissolved in DMSO and dialyzed against MilliQ water (MWCO 3.5 kDa) - after addition of water and NaOH - for 6 days and freeze-dried to afford a white powder.

The general strategy to obtain $PGlu_x$-*b*-βA-PVGLIG-*b*-$PEG_{114}$ copolymers is presented on scheme 3.

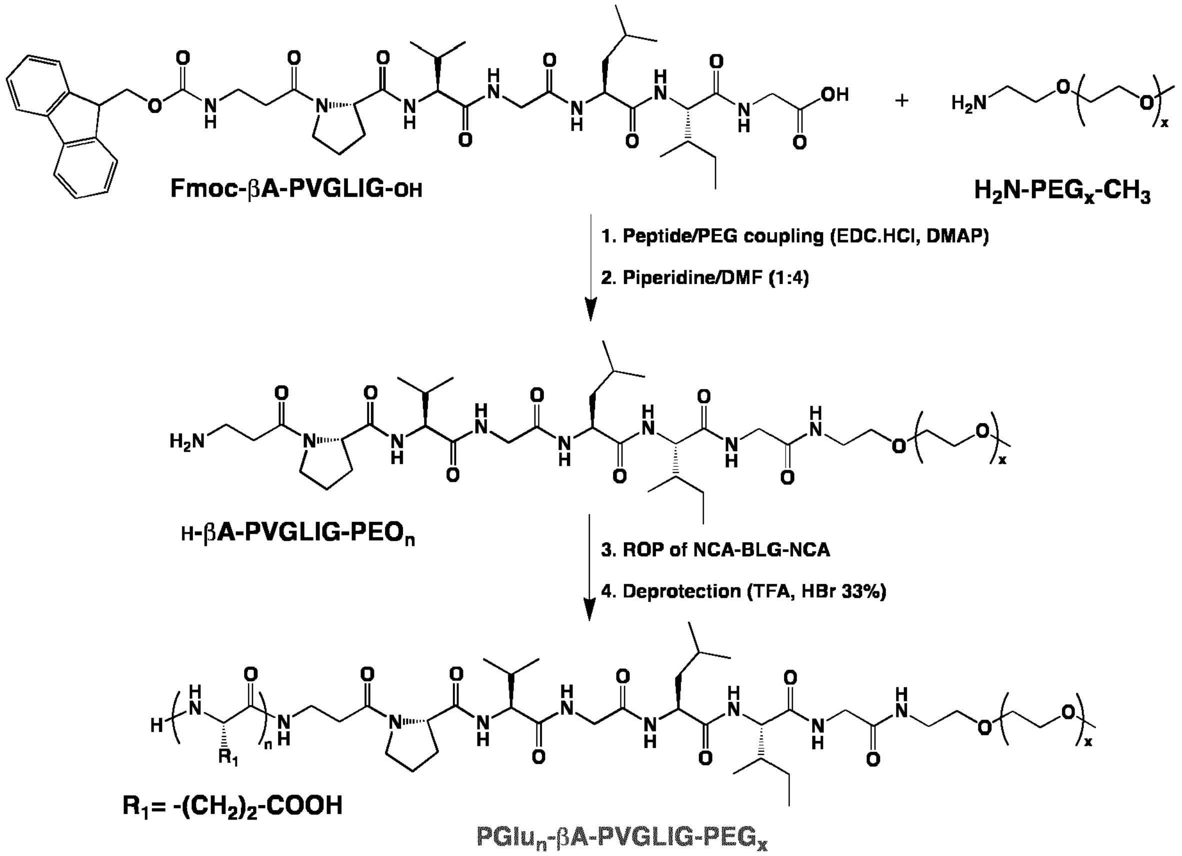


**Scheme 3. Schematic representation of the synthesis of $PGlu_n$-*b*-βA-PVGLIG-*b*-$PEG_{114}$.**

The synthesis of P($Glu_n$-co-$Val_m$)-*b*-βA-PVGLIG-*b*-$PEG_{114}$ copolymers involved the copolymerization of NCA-BLG and NCA-Val during Step 3. The ratio of NCA-BLG to NCA-Val is adapted to relative amount of BLG and Val monomers (n and m) and to the degree of polymerization targeted (n+m). The general strategy to obtain P($Glu_n$-co-$Val_m$)-*b*-βA-PVGLIG-*b*-$PEG_{114}$ copolymers is presented on scheme 4.

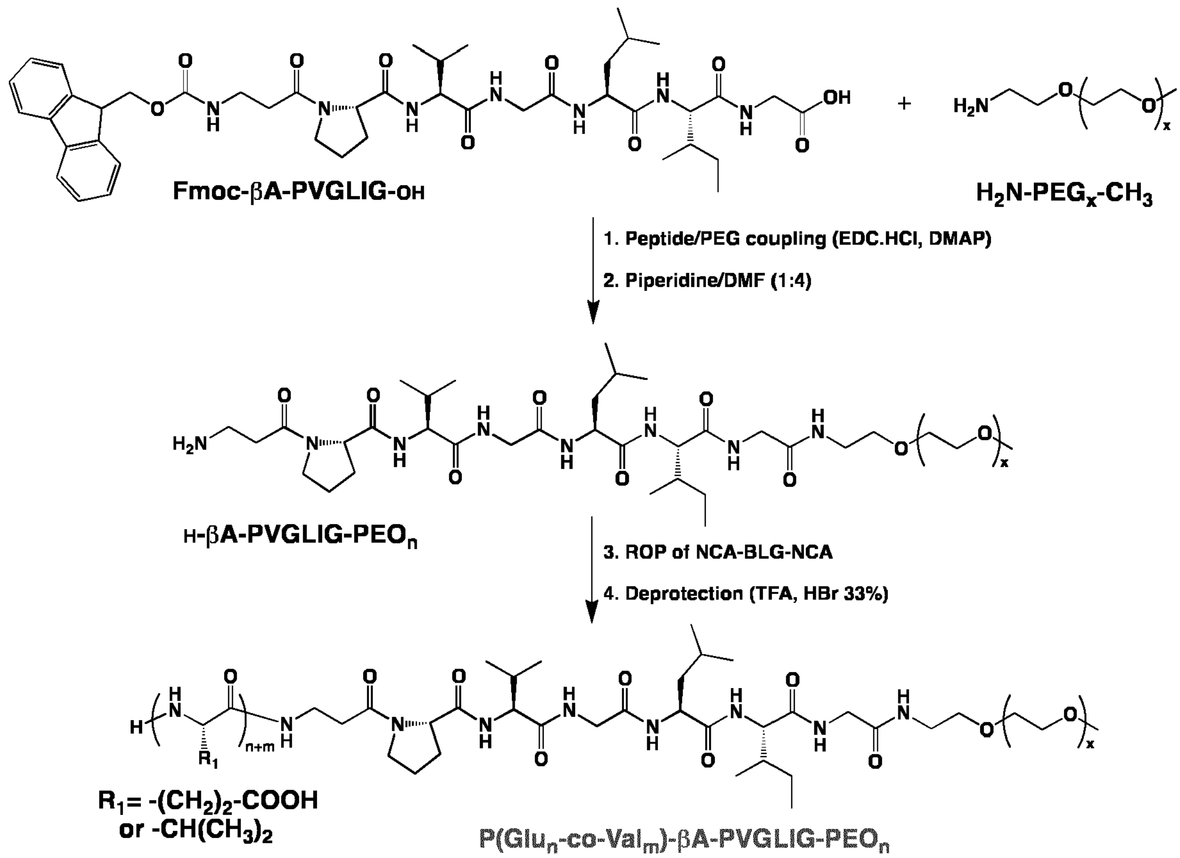


**Scheme 4. Schematic representation of the synthesis of P($Glu_n$-co-$Val_m$)-*b*-βA-PVGLIG-*b*-$PEG_{114}$.**

The following copolymers not being part of the present invention have been prepared by applying the above protocol: $PGlu_{150}$-*b*-βA-PVGLIG-*b*-$PEG_{114}$, $PGlu_{100}$-*b*-βA-PVGLIG-*b*-$PEG_{114}$, $PGlu_{170}$-*b*-βA-PVGLIG-*b*-$PEG_{114}$, P($Glu_{61}$-co-$Val_{16}$)-*b*-βA-PVGLiG-*b*-$PEG_{114}$, and P($Glu_{46}$-co-$Val_{11}$)-*b*-βA-PVGLIG-*b*-$PEG_{114}$.

**USES OF A COPOLYMER OF FORMULA (II) WITH A LINKER**

Example 1: Oil-in-glycerol emulsion by using $PGlu_{150}$-*b*-βA-PVGLIG-*b*-$PEG_{114}$.

| Formulation | | |
|---|---|---|
| | Wt% | Weight (g) |
| $PGlu_{150}$-*b*-βA-PVGLIG-*b*-$PEG_{114}$ | 1 | 0.040 |
| Buffer citrate (pH 5.5) | 89 | 3.560 |
| Octyl Palmitate | 10 | 0.400 |

*Emulsification process*

Polypeptide based block copolymer ($PGlu_{150}$-*b*-βA-PVGLIG-*b*-$PEG_{114}$) was dissolved in buffer citrate at ambient temperature under magnetic stirring (30 minutes, 250 rpm). After that, a pre-emulsion was formed by the addition of octyl palmitate mixture to the water phase. The mixture was agitated (RT, 250 rpm, 30'). Emulsification consisted in the sonication of the mixture using a sonicator UP400S (Hielscher) at 100 % amplitude and 1.0 cycles for 30 min (400 Hz) stirred at 500 rpm. Quantities used are shown in the formulation above.

*Emulsion characterization*

Appearance: the appearance of the final nanocapsules was analysed visually.

Particle size: mean particle size intensity and polydispersity index were measured by Zetasizer Nano ZS (Malvern). Samples were prepared by diluting the emulsion with Octyl Palmitate till a concentration close to 1 wt%. Measurements were carried out in triplicate at 25°C.
Zetasizer Nano Zs: Particle size (nm) = 220 nm; PDI=0.23

Example 2: Oil-in-glycerol emulsion by using $PGlu_{100}$-*b*-βA-PVGLIG-*b*-$PEG_{114}$

| Formulation | | |
|---|---|---|
| | Wt% | Weight (g) |
| $PGlu_{100}$-*b*-βA-PVGLIG-*b*-$PEG_{114}$ | 1 | 0.040 |
| Glycerol | 89 | 3.560 |
| Octyl Palmitate | 10 | 0.400 |

*Emulsification process*

Polypeptide based block copolymer ($PGlu_{100}$-*b*-βA-PVGLIG-*b*-$PEG_{114}$) was dissolved in glycerol at ambient temperature under magnetic stirring (48h, 250 rpm). After that, a pre-emulsion was formed by the addition of octyl palmitate mixture to the water phase. The mixture was agitated (RT, 250 rpm, 30'). Emulsification consisted in the sonication of the mixture using a sonicator UP400S (Hielscher) at 100 % amplitude and 1.0 cycles for 30 min (400 Hz) stirred at 500 rpm. Quantities used are shown in the formulation above.

*Emulsion characterization*

Appearance: the appearance of the final nanocapsules was analysed visually.

Particle size: mean particle size intensity and polydispersity index were measured by Zetasizer Nano ZS (Malvern). Samples were prepared by diluting the emulsion with Octyl Palmitate till a concentration close to 1 wt%. Measurements were carried out in triplicate at 25°C.
Zetasizer Nano Zs: Particle size (nm) = 620 nm; PDI=0.92

Example 3: Oil-in-glycerol emulsion by using $PGlu_{170}$-*b*-βA-PVGLIG-*b*-$PEG_{114}$.

| Formulation | | |
|---|---|---|
| | Wt% | Weight (g) |
| $PGlu_{100}$-*b*-βA-PVGLIG-*b*-$PEG_{114}$ | 1 | 0.040 |
| Buffer citrate (pH 5.5) | 89 | 3.560 |
| Octyl Palmitate | 10 | 0.400 |

*Emulsification process*

Polypeptide based block copolymer ($PGlu_{100}$-*b*-βA-PVGLIG-*b*-$PEG_{114}$) was dissolved in buffer citrate at ambient temperature under magnetic stirring (30 minutes, 250 rpm). After that, a pre-emulsion was formed by the addition of octyl palmitate mixture to the water phase. The mixture was agitated (RT, 250 rpm, 30'). Emulsification consisted in the sonication of the mixture using a sonicator UP400S (Hielscher) at 100 % amplitude and 1.0 cycles for 30 min (400 Hz) stirred at 500 rpm. Quantities used are shown in the formulation above.

*Emulsion characterization*

Appearance: the appearance of the final nanocapsules was analysed visually.

Particle size: mean particle size intensity and polydispersity index were measured by Zetasizer Nano ZS (Malvern). Samples were prepared by diluting the emulsion with Octyl Palmitate till a concentration close to 1 wt%. Measurements were carried out in triplicate at 25°C.
Zetasizer Nano Zs: Particle size (nm) = 180 nm; PDI=0.14

Example 4: Oil-in-glycerol emulsion by using P(Glu$_{61}$-co-Val$_{16}$)-*b*-βA-PVGLIG-*b*-PEG$_{114}$.

| Formulation | | |
|---|---|---|
| | Wt% | Weight (g) |
| P(Glu$_{61}$-co-Val$_{16}$)-*b*-βA-PVGLIG-*b*-PEG$_{114}$ | 1 | 0.040 |
| Buffer citrate (pH 5.5) | 89 | 3.560 |
| Octyl Palmitate | 10 | 0.400 |

*Emulsification process*

Polypeptide based block copolymer (P(Glu$_{61}$-co-Val$_{16}$)-*b*-βA-PVGLIG-*b*-PEG$_{114}$) was dissolved in buffer citrate at ambient temperature under magnetic stirring (30 minutes, 250 rpm). After that, a pre-emulsion was formed by the addition of octyl palmitate mixture to the water phase. The mixture was agitated (RT, 250 rpm, 30'). Emulsification consisted in the sonication of the mixture using a sonicator UP400S (Hielscher) at 100 % amplitude and 1.0 cycles for 30 min (400 Hz) with stirring at 500 rpm. Quantities used are shown in the formulation above.

*Emulsion characterization*

Appearance: the appearance of the final nanocapsules was analysed visually.
Particle size: mean particle size intensity and polydispersity index were measured by Zetasizer Nano ZS (Malvern). Samples were prepared by diluting the emulsion with Octyl Palmitate till a concentration close to 1 wt%. Measurements were carried out in triplicate at 25°C.
Zetasizer Nano Zs: Particle size (nm) = 210 nm; PDI=0.21

Example 5: Oil-in-glycerol emulsion for the encapsulation of Curcuma Longa Root Extract using PGlu$_{150}$-*b*-βA-PVGLIG-*b*-PEG$_{114}$

| Formulation | | |
|---|---|---|
| | Wt% | Weight (g) |
| PGlu$_{150}$-*b*-βA-PVGLIG-*b*-PEG$_{114}$ | 1 | 0.04 |
| Curcuma Longa Root Extract | 7.5 | 0.30 |
| Octyl Palmitate | 2.5 | 0.10 |
| Glycerol | 89 | 3.56 |

*Emulsification process*

Polypeptide based block copolymer (PGlu$_{150}$-*b*-βA-PVGLIG-*b*-PEG$_{114}$) was dissolved in glycerol first at 80°C for 1h and at ambient temperature later for 18 h under magnetic stirring (250 rpm). After that, a pre-emulsion was formed by the addition of octyl palmitate and Curcuma Longa Root Extract (RT, 250 rpm, 10') mixture to the glycerol phase. The mixture was agitated (RT, 250 rpm, 30'). Quantities of each component are shown in the formulation above.
Emulsification consisted in the sonication of the mixture using a sonicator UP400S (Hielscher) at 100 % amplitude and 1.0 cycles for 10 min (400 Hz) stirred at 500 rpm.

*Emulsion characterization*

Appearance: the appearance of the final nanocapsules was analysed visually. Clear viscous good quality emulsion (without sedimentation) was achieved.

Particle size: mean particle size intensity and polydispersity index were measured by Zetasizer Nano ZS (Malvern). Samples were prepared by diluting the emulsion with DDI water till a concentration close to 1 wt%. Measurements were carried out in triplicate at 25°C.
Zetasizer Nano Zs: Particle size (nm) = 290 nm; PDI=0.40

Example 6: Oil-in-glycerol emulsion for the encapsulation of Ceramide NG using $PGlu_{150}$-*b*-βA-PVGLIG-*b*-$PEG_{114}$

| Formulation | | |
|---|---|---|
| | Wt% | Weight (g) |
| $PGlu_{150}$-*b*-βA-PVGLIG-*b*-$PEG_{114}$ | 1 | 0.15 |
| Ceramide NG | 1 | 0.15 |
| Sodium Dilauramidoglutamide Lysine (Pellicer L-30) | 1 | 0.15 |
| Octyldodecanol | 8 | 1.20 |
| Glycerol | 89 | 13.35 |

*Emulsification process*

Polypeptide based block copolymer ($PGlu_{150}$-*b*-βA-PVGLIG-*b*-$PEG_{114}$) was dissolved in glycerol at 80°C for 1h and ambient temperature for 18 h under magnetic stirring (250 rpm). The mixture was further adjusted to 95°C for 10 minutes. On the other hand, Ceramide NG, Sodium Dilauramidoglutamide Lysine and Octyldodecanol were mixed for 10 minutes at 95°C (250 rpm). After that, a pre-emulsion was formed by mixing the octyldodecanol phase to the glycerol phase. The mixture was agitated (95°C, 250 rpm, 20'). Quantities of each component are shown in the formulation above.

Emulsification consisted in the sonication of the mixture using a sonicator UP400S (Hielscher) at 100 % amplitude and 1.0 cycles for 10 min (400 Hz) stirred at 500 rpm and at 60°C (desired temperature reached using an oil bath).

*Emulsion characterization*

Appearance: the appearance of the final nanocapsules was analysed visually. Milky viscous good quality emulsion (without sedimentation) was achieved.

Particle size: mean particle size intensity and polydispersity index were measured by Zetasizer Nano ZS (Malvern). Samples were prepared by diluting the emulsion with DDI water till a concentration close to 1 wt%. Measurements were carried out in triplicate at 25°C.
Zetasizer Nano Zs: Particle size (nm) = 165 nm; PDI=0.06

Example 7: Oil-in-glycerol emulsion for the encapsulation of Retinyl palmitate using $PGlu_{150}$-*b*-βA-PVGLIG-*b*-$PEG_{114}$

| Formulation | | |
|---|---|---|
| | Wt% | Weight (g) |
| $PGlu_{150}$-*b*-βA-PVGLIG-*b*-$PEG_{114}$ | 1 | 0.150 |
| Retinyl palmitate | 7.5 | 1.125 |
| Octyl palmitate | 2.5 | 0.375 |
| Glycerol | 89 | 13.35 |

*Emulsification process*

Polypeptide based block copolymer ($PGlu_{150}$-*b*-βA-PVGLIG-*b*-$PEG_{114}$) was dissolved in glycerol at 80°C for 1h and ambient temperature for 18 h under magnetic stirring (250 rpm). After that, a pre-emulsion was formed by the addition of octyl palmitate and retinyl palmitate mixture (RT, 250 rpm, 10') to the glycerol phase. The mixture was agitated (RT, 250 rpm, 30'). Quantities of each component are shown in the formulation above.

Emulsification consisted in the sonication of the mixture using a sonicator UP400S (Hielscher) at 100 % amplitude and 1.0 cycles for 10 min (400 Hz) stirred at 500 rpm.

*Emulsion characterization*

Appearance: the appearance of the final nanocapsules was analysed visually.

Clear/yellowish viscous good quality emulsion (without sedimentation) was achieved. Particle size: mean particle size intensity and polydispersity index were measured by Zetasizer Nano ZS (Malvern). Samples were prepared by diluting the emulsion with DDI water till a concentration close to 1 wt%. Measurements were carried out in triplicate at 25°C.

Zetasizer Nano Zs: Particle size (nm) = 240 nm; PDI=0.18.

Example 8: Oil-in-glycerol emulsion for the encapsulation of Curcuma Longa Root Extract using P($Glu_{46}$-co-$Val_{11}$)-*b*-βA-PVGLIG-*b*-$PEG_{114}$

| Formulation | | |
|---|---|---|
| | Wt% | Weight (g) |
| P($Glu_{46}$-co-$Val_{11}$)-*b*-βA-PVGLIG-*b*-$PEG_{114}$ | 1 | 1.50 |
| Curcuma Longa Root Extract | 7.5 | 11.25 |
| Octyl Palmitate | 2.5 | 3.75 |
| Glycerol | 89 | 133.50 |

*Emulsification process*

Polypeptide based block copolymer (P($Glu_{46}$-co-$Val_{11}$)-*b*-βA-PVGLIG-*b*-$PEG_{114}$) was dissolved in glycerol at 80°C for 1h and ambient temperature for 18 h under magnetic stirring (250 rpm). After that, a pre-emulsion was formed by the addition of octyl palmitate and Curcuma Longa Root Extract mixture (RT, 250 rpm, 10') to the glycerol phase. The mixture was agitated under mechanical stirring (40°C, 250 rpm, 10'). Quantities of each component are shown in the formulation above.

Emulsification consisted in homogenization of the mixture using a two stage Niro Soavi Panda 2K high pressure homogenizer operating at 450/45 bar pressure for 9 cycles.

*Emulsion characterization*

Appearance: the appearance of the final nanocapsules was analysed visually. Clear viscous good quality emulsion (without sedimentation) was achieved.

Particle size: mean particle size intensity and polydispersity index were measured by Zetasizer Nano ZS (Malvern). Samples were prepared by diluting the emulsion with DDI water till a concentration close to 1 wt%. Measurements were carried out in triplicate at 25°C.

Zetasizer Nano Zs: Particle size (nm) = 220 nm; PDI=0.20.

Example 9: Oil-in-glycerol emulsion for the encapsulation of Ceramide NG using P($Glu_{46}$-co-$Val_{11}$-*b*-βA-PVGLIG-*b*-$PEG_{114}$

| Formulation | | |
|---|---|---|
| | Wt% | Weight (g) |
| P($Glu_{46}$-co-$Val_{11}$)-*b*-βA-PVGLIG-*b*-$PEG_{114}$ | 1 | 0.04 |
| Ceramide NG | 1 | 0.04 |
| Sodium Dilauramidoglutamide Lysine (Pellicer L-30) | 1 | 0.04 |
| Octyldodecanol | 8 | 0.32 |
| Glycerol | 89 | 3.56 |

*Emulsification process*

Polypeptide based block copolymer (P($Glu_{46}$-co-$Val_{11}$)-*b*-βA-PVGLIG-*b*-$PEG_{114}$) was dissolved in glycerol at 80°C for 1 hour and 18 hour at ambient temperature under magnetic stirring (250 rpm). The mixture was further adjusted to 95°C for 10 minutes. On the other hand, Ceramide NG, Sodium Dilauramidoglutamide Lysine and Octyldodecanol were mixed for 10 minutes at 95°C (250 rpm). After that, a pre-emulsion was formed by mixing the octyldodecanol phase to the glycerol phase. The mixture was agitated (95°C, 250 rpm, 20'). Quantities of each component are shown in the formulation above.

Emulsification consisted in the sonication of the mixture using a sonicator UP400S (Hielscher) at 100 % amplitude and 1.0 cycles for 10 min (400 Hz) stirred at 500 rpm and at 60°C (desired temperature reached using an oil bath).

*Emulsion characterization*

Appearance: the appearance of the final nanocapsules was analysed visually. Milky viscous good quality emulsion (without precipitation or sedimentation) was achieved.

Particle size: mean particle size intensity and polydispersity index were measured by Zetasizer Nano ZS (Malvern). Samples were prepared by diluting the emulsion with DDI water till a concentration close to 1 wt%. Measurements were carried out in triplicate at 25°C.

Zetasizer Nano Zs: Particle size (nm) = 190 nm; PDI=0.04.

<u>Example 10:</u> Oil-in-glycerol emulsion for the encapsulation of Retinyl palmitate using P($Glu_{46}$-co-$Val_{11}$)-*b*-βA-PVGLIG-*b*-$PEG_{114}$

| Formulation | | |
|---|---|---|
| | Wt% | Weight (g) |
| P($Glu_{46}$-co-$Val_{11}$)-*b*-βA-PVGLIG-*b*-$PEG_{114}$ | 1 | 0.04 |
| Retinyl palmitate | 7.5 | 0.30 |
| Octyl palmitate | 2.5 | 0.10 |
| Glycerol | 89 | 3.56 |

*Emulsification process*

Polypeptide based block copolymer (P($Glu_{46}$-co-$Val_{11}$)-*b*-βA-PVGLIG-*b*-$PEG_{114}$) was dissolved in glycerol at 80°C for 1h hour first and ambient temperature under magnetic stirring later (18h, 250 rpm). After that, a pre-emulsion was formed by the addition of octyl palmitate and retinyl palmitate mixture (RT, 250 rpm, 10') to the glycerol phase. The mixture was agitated (RT, 250 rpm, 10'). Emulsification consisted in the sonication of the mixture using a sonicator UP400S (Hielscher) at 100 % amplitude and 1.0 cycles for 10 min (400 Hz) stirred at 500 rpm. Quantities of each component are shown in the formulation above.

*Emulsion characterization*

Appearance: the appearance of the final nanocapsules was analysed visually.

Clear/yellowish viscous good quality emulsion (without sedimentation) was achieved. Particle size: mean particle size intensity and polydispersity index were measured by Zetasizer Nano ZS (Malvern). Samples were prepared by diluting the emulsion with DDI water till a concentration close to 1 wt%. Measurements were carried out in triplicate at 25°C.

Zetasizer Nano Zs: Particle size (nm) = 155 nm; PDI=0.10.

**USE OF A CO-SURFACTANT**

The block copolymers not being part of the present invention as described above were used as surfactants for the production of emulsions. The combination of block copolymers and co-stabilizer was demonstrated to be an efficient way to produce water-in-oil and glycerol-in-oil type nanocapsules able to encapsulate hydrophilic active compounds. Different types of co-surfactant (or co-stabilizer) were selected in order to produce stable emulsions (minimum one month without significant changes in particle size).

Emulsification processes were carried out by preparing first a pre-emulsion comprising the surfactant, glycerol, or water, Caprylic/capric triglyceride (MCT) oil and a co-surfactant with low HLB (hydrophilic-lipophilic balance) suitable for water-in-oil emulsions and then emulsifying the mixture by sonication (small scale) or high pressure homogenization (large scale). Experimental conditions are further described in each example.

Particle size and particle size distribution was analysed by Dynamic Light Scattering measurements (Zetasizer Nano ZS). Samples for DLS were prepared by diluting the emulsion with MCT oil at a concentration close to 1 wt%. Measurements were carried out in triplicate at 20°C.

In some examples, additional measurements of particle size were carried out by Laser Diffraction Technique (Malvern Mastersizer).

The concentration of co-surfactant was demonstrated to be a determining parameter in order to achieve emulsions with the desired small size to avoid capsules sedimentation of the glycerol-in-oil emulsions, as presented in Example I, II and III. The best co-surfactants used were:

- EWOCREAM, a polyglyceryl ester with sorbitol and fatty acids derived from Linseed (Linum usitatissimum)(HLB: 5);
- Massocare SMO, sorbitan oleate (HLB 4.3);
- Massacore SMP, sorbitan palmitate (HLB 6.7);
- Polyaldo® 10-10-0, polglyceryl-10 decaoleate (HLB 3); and
- Sorbitan stereate (HLB 4.3).

<u>Example 11:</u> Glycerol-in-oil emulsion with 4.72 wt% Ewocream concentration.

| Formulation | | |
|---|---|---|
| | Wt% | Weight (g) |
| $PEG_{114}$-P($Glu_{46}$-co-$Val_{11}$) | 0.95 | 0.02 |
| Glycerol | 9.43 | 0.20 |
| EWOCREAM | 4.72 | 0.10 |
| MCT oil | 84.90 | 1.8 |

*Emulsification process*

Peptide based block copolymer was dissolved in glycerol at ambient temperature under magnetic stirring overnight (250 rpm). After that, a pre-emulsion was formed by the addition of MCT oil/EWOCREAM (250 rpm, 5 minutes) mixture to the glycerol phase. Pre-emulsion formation was carried out at ambient temperature and under magnetic stirring (250 rpm) for 30 minutes.

Emulsification consisted in the sonication of the mixture using a sonicator UP400S (Hielscher) at 100 % amplitude and 1.0 cycles for 30 min (400 Hz) at 500 rpm. Quantities used are shown in the formulation above.

*Emulsion characterization*

Appearance: the appearance of the final nanocapsules was analysed visually. Milky white good quality emulsion (without precipitation or sedimentation) was achieved.

Particle size: mean particle size intensity and polydispersity index were measured by Zetasizer Nano ZS (Malvern). Samples were prepared by diluting the emulsion with MCT oil till a concentration close to 1 wt%. Measurements were carried out in triplicate at 20°C.

Zetasizer Nano Zs: Particle size (nm) = 260 nm; PDI=0.12
Stability: the stability of the final nanocapsules was analysed visually.
Emulsion was shown to be stable for two weeks.

Example 12: Glycerol-in-oil emulsion with 9.00 wt% Ewocream concentration.

| Formulation | | |
|---|---|---|
| | Wt% | Weight (g) |
| $PEG_{114}$-P($Glu_{46}$-*co*-$Val_{11}$) | 0.90 | 0.02 |
| Glycerol | 9.00 | 0.20 |
| EWOCREAM | 9.00 | 0.20 |
| MCT oil | 81,10 | 1.8 |

*Emulsification process*

Peptide based block copolymer was dissolved in glycerol at ambient temperature under magnetic stirring overnight. After that, a pre-emulsion was formed by the addition of MCT oil/EWOCREAM (250 rpm, 5 minutes mixture to the glycerol phase. Pre-emulsion formation was carried out at ambient temperature and under magnetic stirring (250 rpm) for 30 minutes.

Emulsification consisted in the sonication of the mixture using a sonicator UP400S (Hielscher) at 100 % amplitude and 1.0 cycles for 30 min (400 Hz) at 500 rpm. Quantities used are shown in the formulation above.

*Emulsion characterization*

Appearance: the appearance of the final nanocapsules was analysed visually.

Milky white good quality emulsion (without precipitation or sedimentation) was achieved.

Particle size: mean particle size intensity and polydispersity index were measured by Zetasizer Nano ZS (Malvern). Samples were prepared by diluting the emulsion with MCT oil at a concentration of 1 wt%. Measurements were carried out in triplicate at 20°C.
Zetasizer Nano Zs: Particle size (nm) = 300 nm; PDI=0.2

Stability: the stability of the final nanocapsules was analysed visually.

Emulsion was shown to be stable for two weeks.

Example 13: Glycerol-in-oil emulsion with 16.53 wt% Ewocream concentration.

| Formulation | | |
|---|---|---|
| | Wt% | Weight (g) |
| $PEG_{114}$-P($Glu_{46}$-*co*-$Val_{11}$) | 0.83 | 0.02 |
| Glycerol | 8.26 | 0.20 |
| EWOCREAM | 16.53 | 0.40 |
| MCT oil | 74.38 | 1.8 |

*Emulsification process*

Peptide based block copolymer was dissolved in glycerol at ambient temperature under magnetic stirring overnight. After that, a pre-emulsion was formed by the addition of MCT oil/EWOCREAM (250 rpm, 5 minutes) mixture to the glycerol phase. Pre-emulsion formation was carried out at ambient temperature and under magnetic stirring (250 rpm) for 30 minutes.

Emulsification consisted in the sonication of the mixture using a sonicator UP400S (Hielscher) at 100 % amplitude and 1.0 cycles for 30 min (400 Hz) at 500 rpm. Quantities used are shown in the formulation above.

*Characterization of emulsion*

Appearance: the appearance of the final nanocapsules was analysed visually.

Milky white good quality emulsion (without precipitation or sedimentation) was achieved. Particle size: mean particle size intensity and polydispersity index were measured by Zetasizer Nano ZS (Malvern). Samples were prepared by diluting the emulsion with MCT oil till a concentration close to 1 wt%. Measurements were carried out in triplicate at 20°C. Additionally,

Zetasizer Nano Zs: Particle size (nm) = 150 nm; PDI=0.11
Stability: the stability of the final nanocapsules was analysed visually.
Emulsion presented long term stability (more than 1 month without significant changes on size)

Example 14: Water-in-oil emulsion with 16.53 wt% Ewocream concentration.

| Formulation | | |
|---|---|---|
| | Wt% | Weight (g) |
| $PEG_{114}$-P($Glu_{46}$-*co*-$Val_{11}$) | 0.83 | 0.02 |
| Buffered water at pH=5.5 | 8.26 | 0.20 |
| EWOCREAM | 16.53 | 0.40 |
| MCT oil | 74.38 | 1.8 |

*Emulsification process*

Peptide based block copolymer was dissolved in buffered water at p=5.5 at ambient temperature under magnetic stirring overnight. After that, a pre-emulsion was formed by the addition of MCT oil/EWOCREAM mixture to the glycerol phase. Pre-emulsion formation was carried out at ambient temperature and under magnetic stirring (250 rpm) for 30 minutes.

Emulsification consisted in the sonication of the mixture using a sonicator UP400S (Hielscher) at 100 % amplitude and 1.0 cycles for 30 min (400 Hz). Quantities used are shown in the formulation above.

*Characterization of emulsion*

Appearance: the appearance of the final nanocapsules was analysed visually.

Particle size: mean particle size intensity and polydispersity index were measured by Zetasizer Nano ZS (Malvern). Samples were prepared by diluting the emulsion with MCT oil till a concentration close to 1 wt%. Measurements were carried out in triplicate at 20°C.

Zetasizer Nano Zs: Particle size: 180 nm; PDI: 0.14
Stability: the stability of the final nanocapsules was analysed visually and by DLS measurements
Emulsion presented long term stability (more than 1 month without significant changes on size as judged by DLS measurement)

As demonstrated, the balance between the block copolymer and co-surfactant concentration was a crucial parameter determining for the final size of the capsules. It is worth mentioning that, without the addition of the co-surfactant, emulsions produced by block copolymers alone resulted in very large capsules that show sedimentation but

could be easily redispersed after agitating manually the vial. Moreover, the co-stabilizer alone was not able to form stable and high quality emulsions, even at the highest use concentration (16.67 wt%) (Examples 15 and 16). Hence, the examples exposed demonstrated that the addition of the co-surfactant allow capsules of smaller diameter to be obtained and avoid sedimentation of the capsules, which result in the better looking milky solution.

Example 15: Glycerol-in-oil emulsion with block copolymer as surfactant and without co-stabilizer.

| Formulation | | |
|---|---|---|
| | Wt% | Weight (g) |
| $PEG_{114}$-P($Glu_{46}$-*co*-$Val_{11}$) | 1.20 | 0.048 |
| Glycerol | 8.48 | 0.34 |
| MCT oil | 90.32 | 3.62 |

*Emulsification process*

Peptide based block copolymer was dissolved in glycerol at ambient temperature under magnetic stirring overnight. After that, a pre-emulsion was formed by the addition of MCT oil to the mixture. Pre-emulsion formation was carried out at ambient temperature with magnetic stirring (250 rpm) for 30 minutes.

Emulsification consisted in the sonication of the biphasic mixture using a sonicator UP400S (Hielscher) at 100 % amplitude and 1.0 cycles for 30 min (400 Hz) at 500 rpm. Quantities used are shown in the formulation above.

*Emulsion characterization*

Appearance: the appearance of the final nanocapsules was analysed visually.

Sedimentation observed due to large glycerol droplet
Particle size: 3520 nm PDI: 0.31
Stability: the stability of the final nanocapsules was analysed visually.

Stable for more than a month but sedimentation observed within a few hours. It is important to note that the sedimented capsules could be easily redispersed by gentle handshaking.

Example 16: Glycerol-in-oil emulsion produced without block copolymer as surfactant but only with co-stabilizer (Polyglyceryl ester with sorbitol and fatty acids derived from Linseed (Linum usitatissimum) oil, commercial name: EWOCREAM.

| Formulation | | |
|---|---|---|
| | Wt% | Weight (g) |
| EWOCREAM | 16.67 | 0.4 |
| Glycerol | 8.33 | 0.2 |
| MCT oil | 75.00 | 1.8 |

*Emulsification process*

The co-surfactant and MCT were mixed first at ambient temperature under magnetic stirring (250 rpm) to form the oil phase. Pre-emulsion was further produced by adding glycerol to the mixture in the same agitation conditions for 30 minutes.

Emulsification consisted in the sonication of the biphasic mixture using a sonicator UP400S (Hielscher) at 100 % amplitude and 1.0 cycles for 30 min (400 Hz) at 500 rpm. Quantities used are shown in the formulation above.

*Emulsion characterization*

Appearance: the appearance of the final nanocapsules was analysed visually.

Low quality emulsion with precipitation
Particle size:
Not measured
Stability: the stability of the final nanocapsules was analysed visually.
Not stable (phase separation in less than 24 hours)

The combination of block copolymer and additional co-stabilizer ensured the long term stability of high quality emulsions with different type of block copolymers (Examples 17, 18, and 19).

Example 17: Glycerol-in-oil emulsion with $PEG_{114}$-P($Glu_{46}$-co-$Val_{11}$) peptide based block copolymer.

| Formulation | | |
|---|---|---|
| | Wt% | Weight (g) |
| $PEG_{114}$-P($Glu_{46}$-*co*-$Val_{11}$) | 0.83 | 0.02 |
| Glycerol | 8.26 | 0.20 |
| EWOCREAM | 16.53 | 0.40 |
| MCT oil | 74.38 | 1.8 |

*Emulsification process*

Polypeptide based block copolymer was dissolved in glycerol at ambient temperature under magnetic stirring overnight. After that, a pre-emulsion was formed by the addition of MCT oil/EWOCREAM (250 rpm, 5 minutes) mixture to the glycerol phase. Pre-emulsion formation was carried out at ambient temperature and under magnetic stirring (250 rpm) for 30 minutes.
Emulsification consisted in the sonication of the mixture using a sonicator UP400S (Hielscher) at 100 % amplitude and 1.0 cycles for 30 min (400 Hz) 500 rpm. Quantities used are shown in the formulation above.

*Characterization of emulsion*

Appearance: the appearance of the final nanocapsules was analysed visually.
Milky white good quality emulsion (without precipitation or creaming) was achieved. Particle size: mean particle size intensity and polydispersity index were measured by Zetasizer Nano ZS (Malvern). Samples were prepared by diluting the emulsion with MCT oil till a concentration close to 1 wt%. Measurements were carried out in triplicate at 20°C.

Zetasizer Nano Zs: Particle size (nm) = 90 nm; PDI=0.17
Stability: the stability of the final nanocapsules was analysed visually and by DLS measurements.
Emulsion presented long term stability (more than 1 month without significant changes on size). In fact, particle size after 8 months was 70 nm (PDI=0.24)

Example 18: Glycerol-in-oil emulsion with $PEG_{114}$-$PGlu_{96}$ peptide based block copolymer.

| Formulation | | |
|---|---|---|
| | Wt% | Weight (g) |
| $PEG_{114}$-$PGlu_{96}$ | 0.83 | 0.02 |
| Glycerol | 8.26 | 0.20 |
| EWOCREAM | 16.53 | 0.40 |
| MCT oil | 74.38 | 1800 |

*Emulsification process*

Peptide based block copolymer was dissolved in glycerol at ambient temperature under magnetic stirring overnight. After that, a pre-emulsion was formed by the addition of MCT oil/EWOCREAM (250 rpm, 5 minutes) mixture to the glycerol phase. Pre-emulsion formation was carried out at ambient temperature and under magnetic stirring (250 rpm) for 30 minutes.
Emulsification consisted in the sonication of the mixture using a sonicator UP400S (Hielscher) at 100 % amplitude and 1.0 cycles for 30 min (400 Hz) at 500 rpm. Quantities used are shown in the formulation above.

*Characterization of emulsion*

Appearance: the appearance of the final nanocapsules was analysed visually.
Milky white good quality emulsion (without precipitation or creaming) was achieved. Particle size: mean particle size intensity and polydispersity index were measured by Zetasizer Nano ZS (Malvern). Samples were prepared by diluting the emulsion with MCT oil till a concentration close to 1 wt%. Measurements were carried out in triplicate at 20°C.
Zetasizer Nano Zs: Particle size (nm) = 90 nm; PDI=0.17
Stability: the stability of the final nanocapsules was analysed visually.
Emulsion presented long term stability (more than 1 month without significant changes on appearance).

Example 19: Glycerol-in-oil emulsion with $PEG_{114}$-$PGlu_{151}$ peptide based block copolymer.

| Formulation | | |
|---|---|---|
| | Wt% | Weight (g) |
| $PEG_{114}$-$PGlu_{151}$ | 0.83 | 0.02 |
| Glycerol | 8.26 | 0.20 |
| EWOCREAM | 16.53 | 0.40 |
| MCT oil | 74.38 | 1800 |

*Emulsification process*

Peptide based block copolymer was dissolved in glycerol at ambient temperature under magnetic stirring overnight. After that, a pre-emulsion was formed by the addition of MCT oil/EWOCREAM (250 rpm, 5 minutes) mixture to the glycerol phase. Pre-emulsion formation was carried out at ambient temperature and under magnetic stirring (250 rpm) for 30 minutes.
Emulsification consisted in the sonication of the mixture using a sonicator UP400S (Hielscher) at 100 % amplitude and 1.0 cycles for 30 min (400 Hz) at 550 rpm. Quantities used are shown in the formulation above.

*Characterization of emulsion*

Appearance: the appearance of the final nanocapsules was analysed visually.
Milky white good quality emulsion (without precipitation or creaming) was achieved.
Particle size: mean particle size intensity and polydispersity index were measured by Zetasizer Nano ZS (Malvern). Samples were prepared by diluting the emulsion with MCT oil till a concentration close to 1 wt%. Measurements were carried out in triplicate at 20°C.
Zetasizer Nano Zs: Particle size (nm) = 260 nm; PDI=0.12
Stability: the stability of the final nanocapsules was analysed visually.
Emulsion presented long term stability (more than 1 month without significant changes on appearance).
As mentioned above, different types of co-stabilizers (or co-surfactants) can be used in order to produce oil-in-water and oil-in-glycerol emulsions able to efficiently encapsulate hydrophilic active ingredients. An example can be found in Example 20 below.

Example 20: Water-in-oil emulsion with another co-surfactant: Polyglyceryl-10 decaoleate, commercial name Polyaldo® 10-10-0.

| Formulation | | |
|---|---|---|
| | Wt% | Weight (g) |
| $PEG_{114}$-b-P($Glu_{46}$-*co*-$Val_{11}$) | 1.00 | 0.04 |
| Distilled water | 8.50 | 0.34 |
| POLYALDO 10-10-0 | 16.00 | 0.64 |
| MCT oil | 74.50 | 2.98 |

*Emulsification process*

Peptide based block copolymer was dissolved in distilled water at ambient temperature under magnetic stirring overnight. After that, a pre-emulsion was formed by the addition of MCT oil/POLYALDO 10-10-0 mixture (250 rpm, 5 minutes) to the water phase. Pre-emulsion formation was carried out at ambient temperature and under magnetic stirring (250 rpm) for 30 minutes.

Emulsification consisted in the sonication of the mixture using a sonicator UP400S (Hielscher) at 100 % amplitude and 1.0 cycles for 30 min (400 Hz) at 500 rpm. Quantities used are shown in the formulation above.

*Characterization of emulsion*

Appearance: the appearance of the final nanocapsules was analysed visually.

Milky white good quality emulsion (without precipitation or creaming) was achieved. Particle size: mean particle size intensity and polydispersity index were measured by Zetasizer Nano ZS (Malvern). Samples were prepared by diluting the emulsion with MCT oil till a concentration close to 1 wt%. Measurements were carried out in triplicate at 20°C. Zetasizer Nano Zs: Particle size (nm) = 320 nm; PDI=0.7

Stability: the stability of the final nanocapsules was analysed visually.

Sedimentation was observed after 1 week but the emulsion appeared stable after gentle redispersion by handshaking.

Oil-in-water and oil-in-glycerol emulsions have demonstrated to effectively encapsulate different type of active ingredients. In addition, emulsion production has been demonstrated to be scalable by using high pressure homogenization instead of sonicator. An example of small and large scale production of a formulation that effectively encapsulates Dipotassium Glycyrrhizinate (DPG) is presented in Example 21. A good reproducibility of the scale-up production was demonstrated.

Example 21: Emulsions (small and large scale) emulsions with encapsulated DPG.

| Formulation | | | |
|---|---|---|---|
| | Wt% | Weight (g) small scale | Weight (g) large scale |
| $PEG_{114}$-P($Glu_{46}$-*co*-$Val_{11}$) | 1.00 | 0.15 | 1.50 |
| DPG | 0.75 | 0.11 | 1.12 |
| Glycerol | 8.25 | 1.16 | 11.62 |
| EWOCREAM | 16.00 | 2.40 | 24.00 |
| MCToil | 74.50 | 11.17 | 111.75 |

*Emulsification process*

SMALL SCALE via sonication process

DPG was first dissolved in glycerol at 75°C under magnetic stirring at 250 rpm and for 30 minutes. Once the DPG was properly dissolved, the peptide based block copolymer was added and mixed for an additional 30 minutes.

After that, a pre-emulsion was formed by the addition of MCT oil/EWOCREAM (250 rpm, 5 min) mixture to the glycerol phase. Pre-emulsion formation was carried out at ambient temperature and under magnetic stirring (300 rpm) for 30 minutes.

Emulsification consisted in the sonication of the mixture using a sonicator UP400S (Hielscher) at 100 % amplitude and 1.0 cycles for 30 min (400 Hz) at 500 rpm. Quantities used are shown in the formulation above.

LARGE SCALE via high pressure homogenizer process.

DPG was first dissolved in glycerol at 80°C under magnetic stirring at 250 rpm and for 30 minutes. Once the DPG was properly dissolved, the peptide based block copolymer was added and mixed for an additional hour.

After that, a pre-emulsion was formed by the addition of MCT oil/EWOCREAM mixture (5 minutes, 250 rpm) to the glycerol phase. Pre-emulsion formation was carried out at ambient temperature and under mechanical stirring for 20 minutes.

Emulsification was carried out by homogenization in a two-valves high pressure homogenizer at 45 and 450 bar pressure over 8 cycles. Quantities used are shown in the formulation above.

*Characterization of emulsion*

Appearance: the appearance of the final nanocapsules was analysed visually.

SMALL SCALE: Milky yellow looking good quality emulsion (without precipitation or creaming) was achieved.

LARGE SCALE: Milky yellow looking good quality emulsion (without precipitation or creaming) was achieved.

Particle size: mean particle size intensity and polydispersity index were measured by Zetasizer Nano ZS (Malvern). Samples were prepared by diluting the emulsion with MCT oil till a concentration close to 1 wt%. Measurements were carried out in triplicate at 20°C. Additionally, samples produced at large scale were further characterized using Laser Diffraction (Malvern Mastersizer).

SMALL SCALE

Zetasizer Nano Zs: Particle size (nm) = 140 nm; PDI=0.07

LARGE SCALE

Zetasizer Nano Zs: Particle size (nm) = 160 nm; PDI=0.15
Mastersizer results indicated the presence of a small population of large particles
Stability: the stability of the final nanocapsules was analysed visually and by DLS measurements.
Emulsion presented long term stability (more than 1 month without significant changes on size). In fact, particle size after 3 months was 190 nm (PDI=0.12)

Other natural and sensible actives, such as hyaluronic acid was also successfully encapsulated using the formulation claimed in the present patent (Example 22).

<u>Example 22:</u> Emulsions with encapsulated hyaluronic acid.

| Formulation | | |
|---|---|---|
| | Wt% | Weight (g) |
| $PEG_{114}$-P($Glu_{46}$-*co*-$Val_{11}$) | 2.00 | 0.0960 |
| Hyaluronic acid | 0.10 | 0.0048 |
| Distilled water | 8.40 | 0.4032 |
| EWOCREAM | 16.00 | 0.7680 |
| MCT oil | 74.50 | 3.5280 |

*Emulsification process*

Hyaluronic acid was first dissolved in water at ambient temperature by magnetic agitation for 5 minutes. Peptide

based block copolymer was added to the previous mixture and mixed ambient temperature under magnetic stirring overnight. After that, a pre-emulsion was formed by the addition of MCT oil/Ewocream (5 minutes, 250 rpm) mixture to the water phase. Pre-emulsion formation was carried out at ambient temperature and under magnetic stirring (250 rpm) for 30 minutes.

Emulsification consisted in the sonication of the mixture using a sonicator UP400S (Hielscher) at 100% amplitude and 1.0 cycles for 30 min (400 Hz) at 500 rpm. Quantities used are shown in the formulation above.

*Characterization of emulsion*

Appearance: the appearance of the final nanocapsules was analysed visually.

Almost transparent good quality emulsion (without precipitation or creaming) was achieved.

Particle size: mean particle size intensity and polydispersity index were measured by Zetasizer Nano ZS (Malvern). Samples were prepared by diluting the emulsion with MCT oil till a concentration close to 1 wt%. Measurements were carried out in triplicate at 20°C. In addition, Mastersizer was also used to characterize the samples.
Zetasizer Nano Zs: Particle size (nm) = 110 nm; PDI=0.2

The presence of a large population of higher size particles (more than 10 μm) was observed by laser diffraction.

Stability: the stability of the final nanocapsules was analysed visually.

Emulsion presented good stability for at least 1 week.

Example 23: Glycerol-in-oil emulsion using $PGlu_{150}$-*b*-βA-PVGLIG-*b*-$PEG_{114}$

| Formulation | | |
|---|---|---|
| | Wt% | Weight (g) |
| $PGlu_{150}$-*b*-βA-PVGLIG-*b*-$PEG_{114}$ | 1 | 0.04 |
| Glycerol | 8.5 | 0.34 |
| Ewocream | 16 | 0.64 |
| MCT oil | 74.5 | 2.98 |

*Emulsification process*

Polypeptide based block copolymer ($PGlu_{150}$-*b*-βA-PVGLIG-*b*-$PEG_{114}$) was dissolved in glycerol for 1 hour at 85°C first and at ambient temperature under magnetic stirring later (18h, 250 rpm). After that, a pre-emulsion was formed by the addition of MCT oil/EWOCREAM (250 rpm, 30 minutes) mixture to the glycerol phase. Pre-emulsion formation was carried out at ambient temperature under magnetic stirring (400 rpm) for 2 h. Quantities of each component are shown in the formulation above.

Emulsification consisted in the sonication of the mixture using a sonicator UP400S (Hielscher) at 100 % amplitude and 1.0 cycles for 30 min (400 Hz) stirred at 500 rpm.

*Emulsion characterization*

Appearance: the appearance of the final nanocapsules was analysed visually. Milky white good quality emulsion (without sedimentation) was achieved.

Particle size: mean particle size intensity and polydispersity index were measured by Zetasizer Nano ZS (Malvern). Samples were prepared by diluting the emulsion with MCT oil till a concentration close to 1 wt%. Measurements were carried out in triplicate at 20°C. Zetasizer Nano Zs: Particle size (nm) = 210 nm; PDI=0.4.

Example 24: Glycerol-in-oil emulsion using P($Glu_{46}$-co-$Val_{11}$)-*b*-βA-PVGLIG-*b*-$PEG_{114}$

| Formulation | | |
|---|---|---|
| | Wt% | Weight (g) |
| P($Glu_{46}$-co-$Val_{11}$)-*b*-βA-PVGLIG-*b*-$PEG_{114}$ | 1 | 0.150 |
| Glycerol | 8.5 | 1.275 |
| Ewocream | 16 | 2.400 |
| MCT oil | 74.5 | 11.175 |

*Emulsification process*

The based block copolymer (P($Glu_{46}$-co-$Val_{11}$)-*b*-βA-PVGLIG-*b*-$PEG_{114}$) was dissolved in glycerol for 1 hour at 80°C first and at ambient temperature under magnetic stirring later (18h, 250 rpm). After that, a pre-emulsion was formed by the addition of MCT oil/EWOCREAM (250 rpm, 30 minutes) mixture to the glycerol phase. Pre-emulsion formation was carried out at ambient temperature and under magnetic stirring (250 rpm) for 3 h. Quantities of each component are shown in the formulation above.

Emulsification consisted in the sonication of the mixture using a sonicator UP400S (Hielscher) at 100 % amplitude and 1.0 cycles for 30 min (400 Hz) stirred at 500 rpm.

*Emulsion characterization*

Appearance: the appearance of the final nanocapsules was analysed visually. Milky white good quality emulsion (without sedimentation) was achieved.

Particle size: mean particle size intensity and polydispersity index were measured by Zetasizer Nano ZS (Malvern). Samples were prepared by diluting the emulsion with MCT oil till a concentration close to 1 wt%. Measurements were carried out in triplicate at 20°C.

Zetasizer Nano Zs: Particle size (nm) = 660 nm; PDI=0.60

**EXAMPLES FOR ENCAPSULATION OF ACTIVE INGREDIENTS BASED ON FORMULA (I) WITH CO-SURFACTANT** not being part of the present invention

<u>Example VII</u>: Glycerol-in-oil emulsion for the encapsulation of Dipotassium Glycyrrhizate using $PEG_{114}$-$PGlu_{151}$

| Formulation | | |
|---|---|---|
| | Wt% | Weight (g) |
| $PEG_{114}$-$PGlu_{151}$ | 1 | 0.48 |
| Glycerol | 7.75 | 0.372 |
| Dipotassium Glycyrrhizate | 0.75 | 0.36 |
| Ewocream | 16 | 0.768 |
| MCT oil | 74.5 | 3.576 |

*Emulsification process*

Dipotassium Glycyrrhizate (DPG) was dissolved in glycerol at 80°C for 30 minutes at 250 rpm. Then, polypeptide based block copolymer ($PEG_{114}$-$PGlu_{151}$) was dissolved in the glycerol/DPG mixture for 30 minutes at 85°C first and at ambient temperature under magnetic stirring later (18h, 250 rpm). After that, a pre-emulsion was formed by the addition of MCT oil/EWOCREAM (250 rpm, 30 minutes) mixture to the glycerol phase. Pre-emulsion formation was carried out at ambient temperature and under magnetic stirring (250 rpm) for 30 minutes. Quantities of each component are shown in the formulation above.

Emulsification consisted in the sonication of the mixture using a sonicator UP400S (Hielscher) at 100 % amplitude and 1.0 cycles for 30 min (400 Hz) at 500 rpm.

*Emulsion characterization*

Appearance: the appearance of the final nanocapsules was analysed visually. Milky white good quality emulsion (without sedimentation) was achieved.

Particle size: mean particle size intensity and polydispersity index were measured by Zetasizer Nano ZS (Malvern). Samples were prepared by diluting the emulsion with MCT oil till a concentration close to 1 wt%. Measurements were carried out in triplicate at 20°C. Zetasizer Nano Zs: Particle size (nm) = 210 nm; PDI=0.20.

Example VIII: Water-in-oil emulsion for the encapsulation of Hyaluronic Acid using $PEG_{114}$-$PGlu_{151}$

| Formulation | | |
|---|---|---|
| | Wt% | Weight (g) |
| $PEG_{114}$-$PGlu_{151}$ | 2 | 3.00 |
| DDI water | 8.4 | 12.60 |
| Hyaluronic Acid | 0.1 | 0.15 |
| Ewocream | 16 | 24.00 |
| Isononyl Isononanoate | 73.5 | 110.25 |

*Emulsification process*

Hyaluronic acid was dissolved water at ambient temperature for 10 minutes at 250 rpm. Then, polypeptide based block copolymer ($PEG_{114}$-$PGlu_{151}$) was dissolved in the mixture for 1 hour at ambient temperature. After that, a pre-emulsion was formed by the addition of Isononyl Isononanoate /EWOCREAM (250 rpm, 30 minutes) mixture to the water phase. Pre-emulsion formation was carried out at ambient temperature and under mechanical stirring (500 rpm) for 10 minutes. Quantities of each component are shown in the formulation above.

Emulsification consisted in homogenization of the mixture using a two stage Niro Soavi Panda 2K high pressure homogenizer operating at 450/45 bar pressure for 9 cycles.

*Emulsion characterization*

Appearance: the appearance of the final nanocapsules was analysed visually. Milky white good quality emulsion (without sedimentation) was achieved.

Particle size: mean particle size intensity and polydispersity index were measured by Zetasizer Nano ZS (Malvern). Samples were prepared by diluting the emulsion with Isononyl Isononanoate till a concentration close to 1 wt%. Measurements were carried out in triplicate at 20°C.

Zetasizer Nano Zs: Particle size (nm) = 120 nm; PDI=0.1.

**EXAMPLES FOR ENCAPSULATION OF ACTIVE INGREDIENTS BASED ON FORMULA (II) WITH CO-SURFACTANT** not being part of the present invention

Example XV: Glycerol-in-oil emulsion for the encapsulation of Dipotassium Glycyrrhizate using $PGlu_{150}$-*b*-βA-PVGLIG-*b*-$PEG_{114}$

| Formulation | | |
|---|---|---|
| | Wt% | Weight (g) |
| $PGlu_{150}$-*b*-βA-PVGLIG-*b*-$PEG_{114}$ | 1 | 2.0 |
| Glycerol | 7.75 | 15.5 |
| Dipotassium Glycyrrhizate | 0.75 | 1.5 |

(continued)

| Formulation | | |
|---|---|---|
| | Wt% | Weight (g) |
| Ewocream | 16 | 32.0 |
| MCT oil | 74.5 | 149.0 |

*Emulsification process*

Dipotassium Glycyrrhizate (DPG) was dissolved in glycerol at 80°C for 30 minutes at 250 rpm. Then, polypeptide based block copolymer (PGlu$_{150}$-*b*-βA-PVGLIG-*b*-PEG$_{114}$) was dissolved in the glycerol/DPG mixture for 1 hour at 85°C first and at ambient temperature under magnetic stirring later (18h, 250 rpm). After that, a pre-emulsion was formed by the addition of MCT oil/EWOCREAM (250 rpm, 30 minutes) mixture to the glycerol phase. Pre-emulsion formation was carried out at 85°C and under mechanical stirring (250 rpm) for 15 minutes. Quantities of each component are shown in the formulation above. Emulsification consisted in homogenization of the mixture using a two stage Niro Soavi Panda 2K high pressure homogenizer operating at 450/45 bar pressure for 9 cycles.

*Emulsion characterization*

Appearance: the appearance of the final nanocapsules was analysed visually. Milky white good quality emulsion (without sedimentation) was achieved.
Particle size: mean particle size intensity and polydispersity index were measured by Zetasizer Nano ZS (Malvern). Samples were prepared by diluting the emulsion with MCT oil till a concentration close to 1 wt%. Measurements were carried out in triplicate at 20°C.
Zetasizer Nano Zs: Particle size (nm) = 150 nm; PDI=0.10

Example XVI: Water-in-oil emulsion for the encapsulation of Hyaluronic Acid using PGlu$_{150}$-*b*-βA-PVGLIG-*b*-PEG$_{114}$

| Formulation | | |
|---|---|---|
| | Wt% | Weight (g) |
| PGlu$_{150}$-*b*-βA-PVGLIG-*b*-PEG$_{114}$ | 2 | 3.00 |
| DDI water | 8.4 | 12.60 |
| Hyaluronic Acid | 0.1 | 0.15 |
| Ewocream | 16 | 24.00 |
| Isononyl Isononanoate | 73.5 | 110.25 |

*Emulsification process*

Hyaluronic acid was dissolved water at ambient temperature for 10 minutes at 250 rpm. Then, polypeptide based block copolymer (PGlu$_{150}$-*b*-βA-PVGLIG-*b*-PEG$_{114}$) was dissolved in the mixture for 18 hours at ambient temperature. After that, a pre-emulsion was formed by the addition of Isononyl Isononanoate/EWOCREAM (250 rpm, 30 minutes) mixture to the water phase. Pre-emulsion formation was carried out at ambient temperature and under mechanical stirring (500 rpm) for 10 minutes. Quantities of each component are shown in the formulation above.
Emulsification consisted in homogenization of the mixture using a two stage Niro Soavi Panda 2K high pressure homogenizer operating at 450/45 bar pressure for 9 cycles.

*Emulsion characterization*

Appearance: the appearance of the final nanocapsules was analysed visually. Milky white good quality emulsion (without sedimentation) was achieved.
Particle size: mean particle size intensity and polydispersity index were measured by Zetasizer Nano ZS (Malvern). Samples were prepared by diluting the emulsion with Isononyl Isononanoate till a concentration close to 1 wt%. Measurements were carried out in triplicate at 20°C.
Zetasizer Nano Zs: Particle size (nm) = 110 nm; PDI=0.09

Example XVII: Glycerol-in-oil emulsion for the encapsulation of Dipotassium Glycyrrhizate using P($Glu_{46}$-co-$Val_{11}$)-*b*-βA-PVGLIG-*b*-$PEG_{114}$

| Formulation | | |
|---|---|---|
| | Wt% | Weight (g) |
| P($Glu_{46}$-co-$Val_{11}$)-*b*-βA-PVGLIG-*b*-$PEG_{114}$ | 1 | 2.0 |
| Glycerol | 7.75 | 15.5 |
| Dipotassium Glycyrrhizate | 0.75 | 1.5 |
| Ewocream | 16 | 32.0 |
| MCT oil | 74.5 | 149.0 |

*Emulsification process*

Dipotassium Glycyrrhizate (DPG) was dissolved in glycerol at 80°C for 30 minutes at 250 rpm. Then, polypeptide based block copolymer (P($Glu_{46}$-co-$Val_{11}$)-*b*-βA-PVGLIG-*b*-$PEG_{114}$) was dissolved in the glycerol/DPG mixture for 1 hour at 80°C first and at ambient temperature under magnetic stirring later (18h, 250 rpm). After that, a pre-emulsion was formed by the addition of MCT oil/EWOCREAM (250 rpm, 30 minutes) mixture to the glycerol phase. Pre-emulsion formation was carried out at 85°C and under mechanical stirring (250 rpm) for 15 minutes. Quantities of each component are shown in the formulation above.

Emulsification consisted in homogenization of the mixture using a two stage Niro Soavi Panda 2K high pressure homogenizer operating at 450/45 bar pressure for 9 cycles.

*Emulsion characterization*

Appearance: the appearance of the final nanocapsules was analysed visually. Milky white good quality emulsion (without sedimentation) was achieved.

Particle size: mean particle size intensity and polydispersity index were measured by Zetasizer Nano ZS (Malvern). Samples were prepared by diluting the emulsion with MCT oil till a concentration close to 1 wt%. Measurements were carried out in triplicate at 20°C.
Zetasizer Nano Zs: Particle size (nm) = 160 nm; PDI=0.05

Example XVIII: Water-in-oil emulsion for the encapsulation of Hyaluronic Acid using P($Glu_{46}$-co-$Val_{11}$)-*b*-βA-PVGLIG-*b*-$PEG_{114}$

| Formulation | | |
|---|---|---|
| | Wt% | Weight (g) |
| P($Glu_{46}$-co-$Val_{11}$)-*b*-βA-PVGLIG-*b*-$PEG_{114}$ | 2 | 3.00 |
| DDI water | 8.4 | 12.60 |
| Hyaluronic Acid | 0.1 | 0.15 |
| Ewocream | 16 | 24.00 |
| Isononyl Isononanoate | 73.5 | 110.25 |

*Emulsification process*

Hyaluronic acid was dissolved water at ambient temperature for 10 minutes at 250 rpm. Then, polypeptide based block copolymer (P($Glu_{46}$-co-$Val_{11}$)-*b*-βA-PVGLIG-*b*-$PEG_{114}$) was dissolved in the mixture for 18 hours at ambient temperature. After that, a pre-emulsion was formed by the addition of Isononyl Isononanoate/EWOCREAM (250 rpm, 30 minutes) mixture to the water phase. Pre-emulsion formation was carried out at ambient temperature and under mechanical stirring (500 rpm) for 10 minutes. Quantities of each component are shown in the formulation above.

Emulsification consisted in homogenization of the mixture using a two stage Niro Soavi Panda 2K high pressure homogenizer operating at 450/45 bar pressure for 9 cycles.

*Emulsion characterization*

Appearance: the appearance of the final nanocapsules was analysed visually. Milky white good quality emulsion (without sedimentation) was achieved.

Particle size: mean particle size intensity and polydispersity index were measured by Zetasizer Nano ZS (Malvern). Samples were prepared by diluting the emulsion with Isononyl Isononanoate till a concentration close to 1 wt%. Measurements were carried out in triplicate at 20°C.
Zetasizer Nano Zs: Particle size (nm) = 115 nm; PDI=0.14

## Claims

1. Use of a copolymer having the following formula (II):

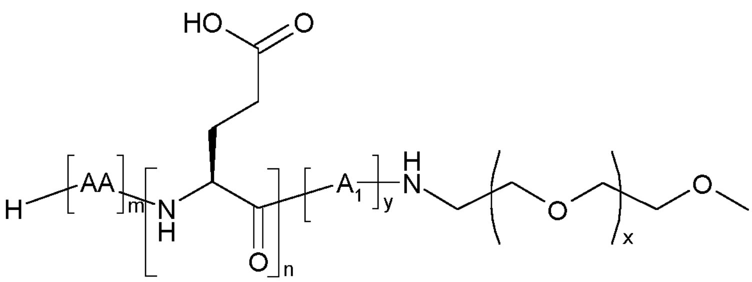

(II)

wherein:

- $A_1$ is a linker, preferably an enzyme cleavable peptide sequence, and y is 0 or 1;
- AA is a residue of a hydrophobic amino acid, such as valine, leucine, isoleucine, or phenylalanine;
- x is an integer comprised between 17 and 270, and preferably between 17 and 114;
- n is an integer comprised between 5 and 170; and
- m is an integer comprised between 0 and 80,

optionally in the presence of a co-surfactant,
as surfactant for the stabilization of an emulsion at a pH comprised between 4 and 6.5, preferably between 5 and 6.

2. The use of claim 1, wherein y is 1.

3. The use of claim 1 or 2, wherein $A_1$ is an enzyme-cleavable peptide sequence, in particular cleavable by matrix metalloproteinase (MMP), in particular by MMP2 or MMP9; particularly,
$A_1$ is a peptide comprising from 4 to 10 amino acids, preferably from 4 to 7 amino acids, and containing in particular at least one glycine residue, and preferably at least two glycine residues, and optionally at least one further amino acid in the β configuration such as beta-alanine; more particularly $A_1$ is chosen from the group consisting of the following peptides: βA-PVGLIG, βA-GFLG, βA-GRFG, and βA-GFKFLG.

4. The use of claim 1, of a copolymer of formula (II) wherein when y is 0, then copolymer is one having the following formula (I):

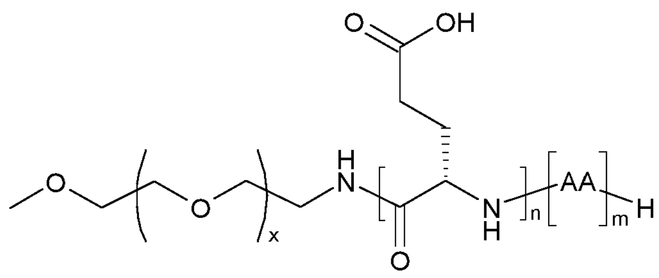

(I)

wherein AA, x, m and n are as defined in claim 1.

5. The use of any one of claims 1 to 4 wherein y=1, $A_1$ is βA-PVGLIG, m is 0, n is 100, 150, or 170 and x is 114; or, alternatively, wherein y=1, $A_1$ is βA-PVGLIG, m is 16, n is 61 and x is 114; or, alternatively, wherein y=1, $A_1$ is βA-PVGLIG, m is 11, n is 46 and x is 114; or, alternatively, y=0; m is 0, n is 96, 100, or 151 and x is 114; or, alternatively, y=0, m is 5, n is 16 and x is 114; or, alternatively, y=0, m is 11, n is 44 and x is 114; or, alternatively, wherein y=0 m is 11, n is 46 and x is 114.

6. The use according to any one of claims 1 to 5, which comprises the addition of a co-surfactant, particularly a co-surfactant having an hydrophilic-lipophilic balance (HLB) below 10.

**Patentansprüche**

1. Verwendung eines Copolymers mit der folgenden Formel (II):

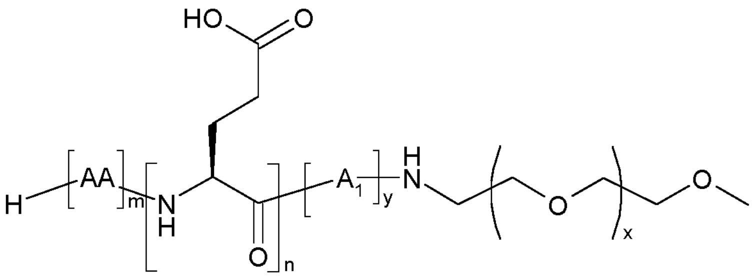

(II)

wobei:

- $A_1$ ein Linker, vorzugsweise eine enzymabspaltbare Peptidsequenz, ist und y = 0 oder 1 ist;
- AA ein Rest einer hydrophoben Aminosäure, wie etwa Valin, Leucin, Isoleucin oder Phenylalanin ist;
- x eine ganze Zahl zwischen 17 und 270 und vorzugsweise zwischen 17 und 114 ist;
- n eine ganze Zahl zwischen 5 und 170 ist; und
- m eine ganze Zahl zwischen 0 und 80 ist,

gegebenenfalls in Gegenwart eines Co-Tensids,
als Tensid zur Stabilisierung einer Emulsion bei einem pH-Wert zwischen 4 und 6,5, vorzugsweise zwischen 5 und 6.

2. Die Verwendung von Anspruch 1, wobei y = 1 ist.

3. Die Verwendung von Anspruch 1 oder 2, wobei $A_1$ eine enzymspaltbare Peptidsequenz ist, insbesondere spaltbar durch eine Matrix-Metalloproteinase (MMP), insbesondere durch MMP2 oder MMP9; insbesondere $A_1$ ein Peptid ist, das von 4 bis 10 Aminosäuren, vorzugsweise von 4 bis 7 Aminosäuren, umfasst und insbesondere mindestens einen Glycinrest, und vorzugsweise mindestens zwei Glycinreste, und gegebenenfalls mindestens eine weitere Aminosäure in der β-Konfiguration, wie beta-Alanin, enthält; insbesondere $A_1$ ausgewählt ist aus der Gruppe bestehend aus den folgenden Peptiden: βA-PVGLIG, βA-GFLG, βA-GRFG und βA-GFKFLG.

4. Die Verwendung von Anspruch 1 eines Copolymers der Formel (II), wobei, wenn y = 0 ist, das Copolymer eines mit der folgenden Formel **(I)** ist:

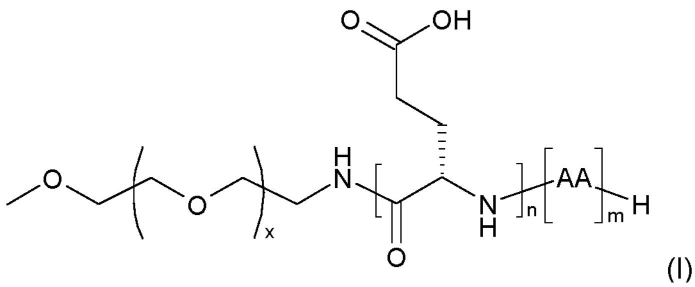

(I)

wobei AA, x, m und n wie in Anspruch 1 definiert sind.

5. Die Verwendung von einem der Ansprüche 1 bis 4, wobei y = 1, $A_1$ βA-PVGLIG ist, m = 0 ist, n = 100, 150 oder 170 ist und x = 114 ist; oder, alternativ, wobei y = 1 ist, $A_1$ βA-PVGLIG ist, m = 16 ist, n = 61 ist und x = 114 ist; oder, alternativ, wobei y = 1 ist, $A_1$ βA-PVGLIG ist, m = 11 ist, n = 46 ist und x = 114 ist; oder, alternativ, y = 0 ist; m = 0 ist, n = 96, 100 oder 151 ist und x = 114 ist; oder alternativ, y = 0 ist, m = 5 ist, n = 16 ist und x = 114 ist; oder alternativ, y = 0 ist, m = 11 ist, n = 44 ist und x = 114 ist; oder, alternativ, wobei y = 0 ist, m = 11 ist, n = 46 ist und x = 114 ist.

6. Die Verwendung nach einem der Ansprüche 1 bis 5, welche die Zugabe eines Co-Tensids, insbesondere eines Co-Tensids mit einem hydrophilenlipophilen Gleichgewicht (HLB) unter 10, umfasst.

**Revendications**

1. Utilisation d'un copolymère ayant la formule (II) suivante :

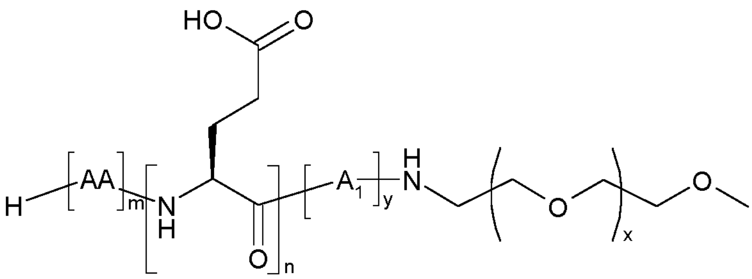

(II)

dans laquelle :

- $A_1$ est un lieur, de préférence une séquence peptidique clivable par enzyme, et y est 0 ou 1 ;
- AA est un reste d'un acide aminé hydrophobe, tel que la valine, la leucine, l'isoleucine ou la phénylalanine ;
- x est un nombre entier compris entre 17 et 270, et de préférence entre 17 et 114 ;
- n est un nombre entier compris entre 5 et 170 ; et
- m est un nombre entier compris entre 0 et 80,

éventuellement en présence d'un co-tensioactif,
comme tensioactif pour la stabilisation d'une émulsion à un pH compris entre 4 et 6,5, de préférence entre 5 et 6.

2. L'utilisation de la revendication 1, dans laquelle y est 1.

3. L'utilisation de la revendication 1 ou 2, dans laquelle $A_1$ est une séquence peptidique clivable par enzyme, en particulier clivable par une métalloprotéinase matricielle (MMP), en particulier par MMP2 ou MMP9 ; en particulier, $A_1$ est un peptide comprenant de 4 à 10 acides aminés, de préférence de 4 à 7 acides aminés, et contenant en particulier au moins un reste de glycine, et de préférence au moins deux restes de glycine, et éventuellement au moins un autre acide aminé dans la configuration β tel que la bêta-alanine ; plus particulièrement $A_1$ est choisi dans le groupe constitué par les peptides suivants : βA-PVGLIG, βA-GFLG, βA-GRFG, et βA-GFKFLG.

4. L'utilisation de la revendication 1 d'un copolymère de formule (II) dans laquelle, lorsque y est 0, alors le copolymère est un copolymère ayant la formule (I) suivante :

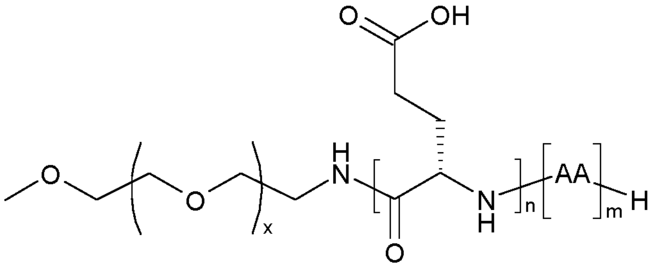

(I)

dans laquelle AA, x, m et n sont tels que définis dans la revendication 1.

5. L'utilisation de l'une quelconque des revendications 1 à 4, dans laquelle y = 1, $A_1$ est βA-PVGLIG, m est 0, n est 100, 150 ou 170 et x est 114 ; ou, en variante, dans laquelle y = 1, $A_1$ est βA-PVGLIG, m est 16, n est 61 et x est 114 ; ou, en variante, dans laquelle y = 1, $A_1$ est βA-PVGLIG, m est 11, n est 46 et x est 114 ; ou, en variante, y = 0 ; m est 0, n est 96, 100 ou 151 et x est 114 ; ou, en variante, y = 0, m est 5, n est 16 et x est 114 ; ou, en variante, y = 0, m est 11, n est 44 et x est 114 ; ou, en variante, dans laquelle y = 0, m est 11, n est 46 et x est 114.

6. L'utilisation selon l'une quelconque des revendications 1 à 5, laquelle comprend l'addition d'un co-tensioactif, en particulier un co-tensioactif ayant un équilibre hydrophile-lipophile (HLB) inférieur à 10.

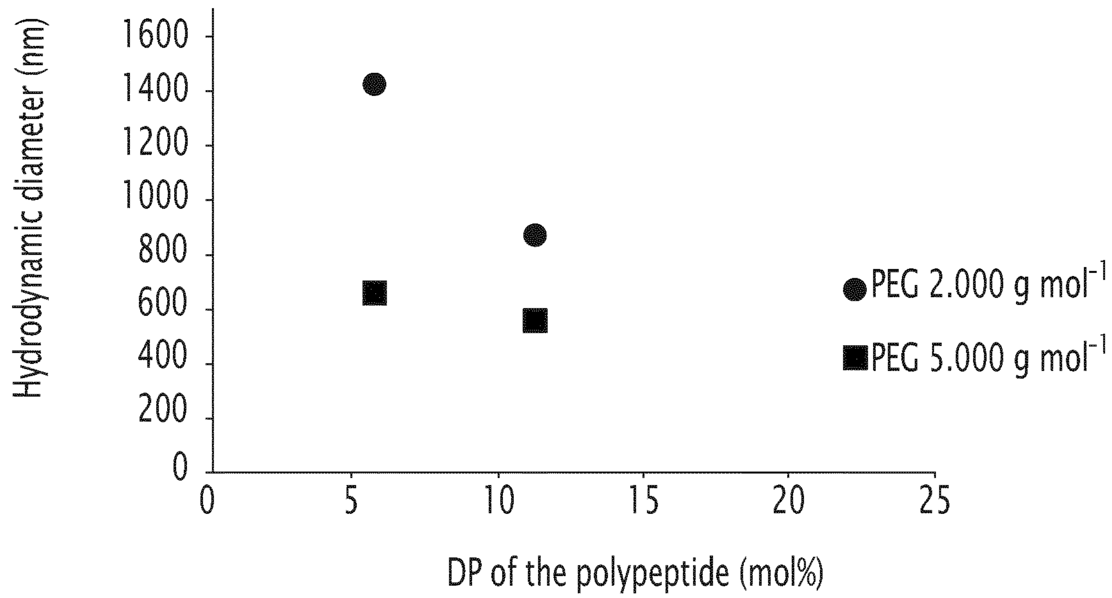
Hydrodynamic diameter (nm)
1600
1400
1200
1000
800
600
400
200
0
0
5
10
15
20
25
DP of the polypeptide (mol%)
PEG 2.000 g mol-1
PEG 5.000 g mol-1

FIG.1

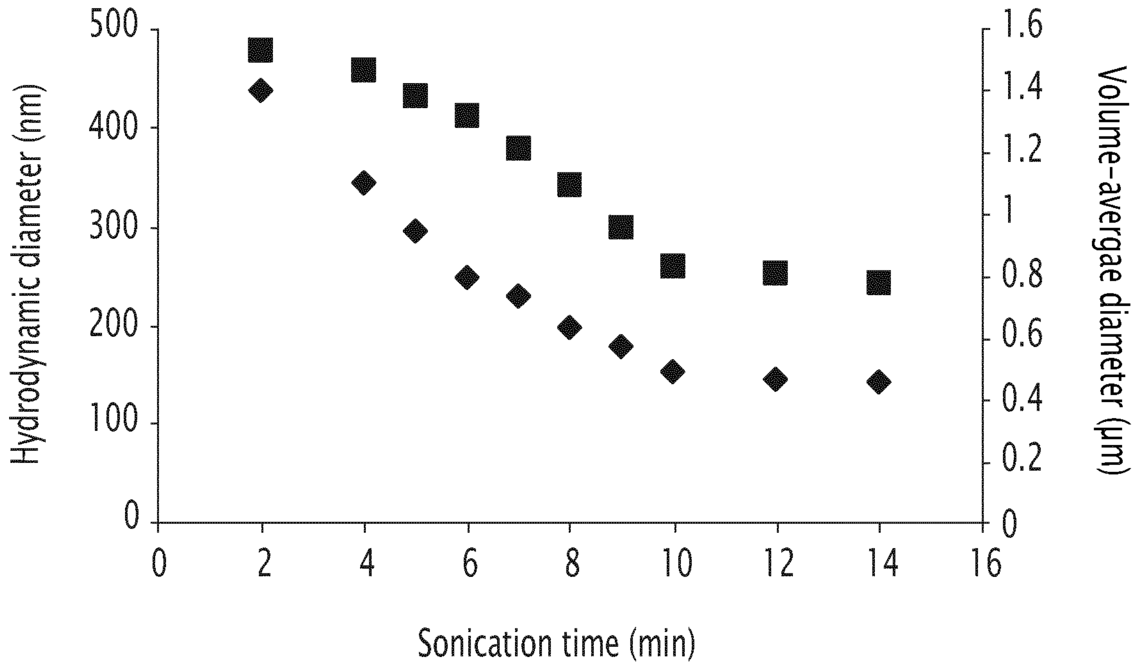
Hydrodynamic diameter (nm)
500
400
300
200
100
0
0
2
4
6
8
10
12
14
16
Sonication time (min)
1.6
1.4
1.2
1
0.8
0.6
0.4
0.2
0
Volume-avergae diameter (μm)

FIG.2

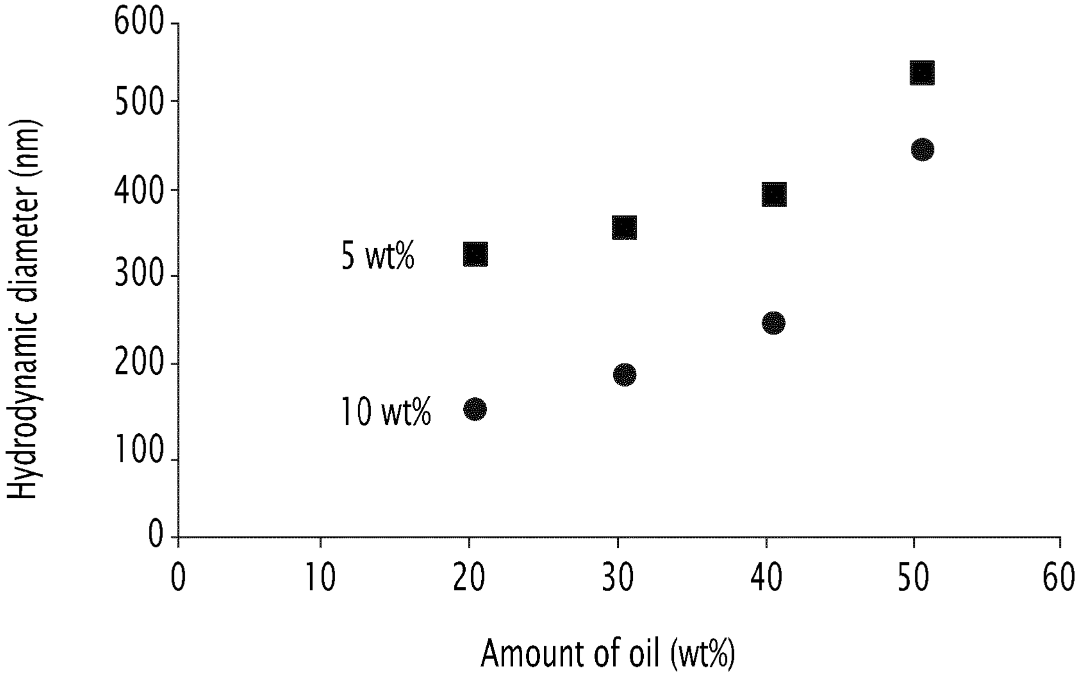
Hydrodynamic diameter (nm)
600
500
400
300
200
100
0
5 wt%
10 wt%
0
10
20
30
40
50
60
Amount of oil (wt%)

FIG.3

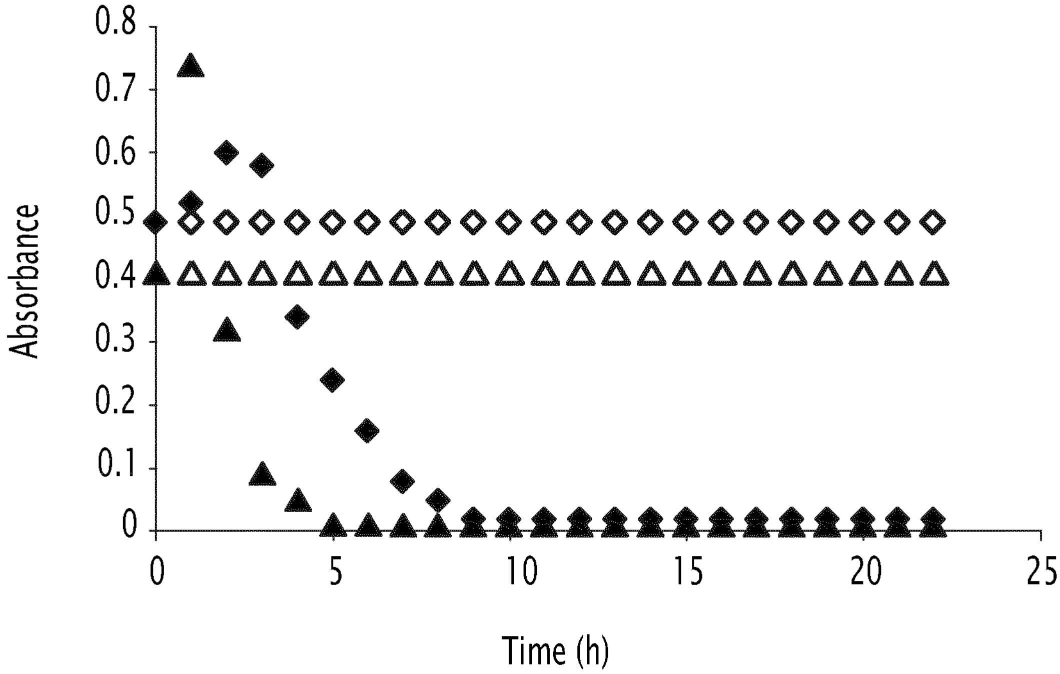
Absorbance
0.8
0.7
0.6
0.5
0.4
0.3
0.2
0.1
0
0
5
10
15
20
25
Time (h)

FIG.4

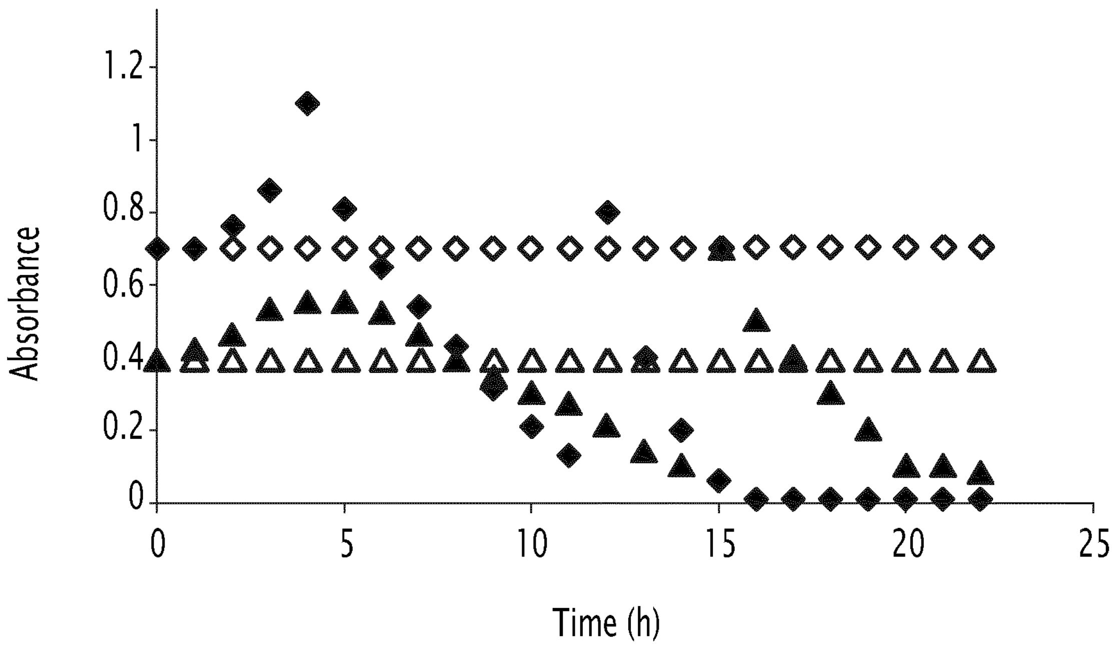


FIG.5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **LV SHIXIAN et al.** Co-delivery of doxorubicin and paclitaxel by PEG-polypeptide nanovehicle for the treatment of non-small cell lung cancer. *Biomaterials,* 2014, vol. 35 (23), 6118-6129
- *J. Am. Chem. Soc.,* 2006, vol. 128, 6540-6541
- *Nature,* 2008, vol. 455, 85
- *J. Am. Chem. Soc.,* 2006, vol. 728, 6540-6541